(19) Europäisches Patentamt
European Patent Office
Office européen des brevets

(11) **EP 2 849 694 B1**

(12) **EUROPÄISCHE PATENTSCHRIFT**

(45) Veröffentlichungstag und Bekanntmachung des Hinweises auf die Patenterteilung:
**28.02.2018 Patentblatt 2018/09**

(21) Anmeldenummer: **13719119.3**

(22) Anmeldetag: **30.04.2013**

(51) Int Cl.:
*A61F 13/58* *(2006.01)*   *A61F 13/49* *(2006.01)*
*A61F 13/496* *(2006.01)*   *A61F 13/15* *(2006.01)*

(86) Internationale Anmeldenummer:
**PCT/EP2013/058976**

(87) Internationale Veröffentlichungsnummer:
**WO 2013/171065 (21.11.2013 Gazette 2013/47)**

(54) **INKONTINENZARTIKEL IN HÖSCHENFORM**

INCONTINENCE ARTICLE IN THE FORM OF BRIEFS

ARTICLE POUR INCONTINENCE EN FORME DE CULOTTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.05.2012   DE 102012208392**

(43) Veröffentlichungstag der Anmeldung:
**25.03.2015   Patentblatt 2015/13**

(73) Patentinhaber: **Paul Hartmann AG**
**89522 Heidenheim (DE)**

(72) Erfinder:
• **GASSNER, Oliver**
  **50678 Köln (DE)**
• **BEYRLE, Andreas**
  **89564 Nattheim (DE)**

(74) Vertreter: **DREISS Patentanwälte PartG mbB**
**Friedrichstrasse 6**
**70174 Stuttgart (DE)**

(56) Entgegenhaltungen:
EP-A1- 2 241 296       EP-A1- 2 377 500
WO-A1-2011/055546       DE-A1-102007 056 126

**Beschreibung**

Die Erfindung betrifft einen Inkontinenzartikel in Höschenform für die Aufnahme von Körperausscheidungen mit einem vorderen Bauchabschnitt und einem hinteren Rückenabschnitt mit vliesstoffbasierten oder vliesstoffhaltigen Materialien oder Verbundmaterialien, die von separaten und in einer Längsrichtung entlang einer Längsmittelachse voneinander beabstandeten Komponenten gebildet sind, jedoch zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, und mit einem einen Absorptionskörper aufweisenden Schrittabschnitt, der sich in der Längsrichtung zwischen Bauchabschnitt und Rückenabschnitt erstreckt und mit seiner körperabgewandten Seite an den Bauchabschnitt und an den Rückenabschnitt in einem jeweiligen Überlappungsbereich unlösbar angefügt ist, wobei der Bauchabschnitt, der Rückenabschnitt und der Schrittabschnitt gemeinsam Beinöffnungen des Inkontinenzartikels begrenzen, wobei in dem Bauchabschnitt und dem Rückenabschnitt erste Elastifizierungsmittel vorgesehen sind, die sich in einem Abstand voneinander und parallel zueinander in Quer- oder Hüftumfangsrichtung erstrecken und so den Bauchabschnitt und den Rückenabschnitt flächenhaft elastifizieren, wobei in einem schrittseitigen und den Beinöffnungen zugewandten Bereich des Bauchabschnitts und des Rückenabschnitts zweite Elastifizierungsmittel vorgesehen sind, die sich insbesondere ausgehend von den beiden Seitennahtbereichen in Richtung auf eine Längsmittelachse des Inkontinenzartikels erstrecken und sich dabei bis in den Überlappungsbereich von Schrittabschnitt und Bauchabschnitt (im folgenden auch vorderer Überlappungsbereich genannt) bzw. von Schrittabschnitt und Rückenabschnitt (im folgenden auch hinterer Überlappungsbereich genannt) erstrecken, wobei sie dort ihrer elastifizierenden Wirkung benommen sein können, insbesondere geschnitten sein können. Derartige Inkontinenzartikel sind bekannt aus DE 10 2007 056 126 A1, EP 2 241 296 A1, WO 2011/055546 A1 (jedoch ohne Offenbarung von Vliesstoff als Bauch- oder Rückenabschnitt) und EP 2 377 500 A1.

Es handelt sich also um einen dreikomponentigen Inkontinenzartikel, wobei der Bauchabschnitt, der Rückenabschnitt und der Schrittabschnitt diese drei Komponenten bilden. Der Bauchabschnitt und der Rückenabschnitt sowie der Schrittabschnitt werden als voneinander separate Komponenten einer Herstellungsvorrichtung zugeführt bzw. darin gefördert. Dabei sind die Komponenten typischerweise in einer jeweiligen Förderebene in einem flach oder eben ausgebreiteten Zustand geführt. Dabei werden der Bauchabschnitt und der Rückenabschnitt in der späteren Querrichtung des Inkontinenzartikels gefördert; sie sind dabei in der späteren Längsrichtung des Inkontinenzartikels voneinander beabstandet geführt. Somit verläuft die spätere Quer- oder Hüftumfangsrichtung des Inkontinenzartikels in der Maschinenrichtung der Herstellungsvorrichtung. Der vorgenannte Abstand zwischen Bauchabschnitt und Rückenabschnitt wird dann durch Aufbringen des Schrittabschnitts als dritter Komponente gewissermaßen überbrückt, wobei ein Überlappungsbereich zwischen Schrittabschnitt und Bauchabschnitt und zwischen Schrittabschnitt und Rückenabschnitt gebildet wird, wobei die drei Komponenten in dem jeweiligen Überlappungsbereich unlösbar miteinander gefügt werden. Schließlich werden der Bauchabschnitt und der Rückenabschnitt wie vorstehend erwähnt an beidseitigen Seitennahtbereichen miteinander verbunden. Ein derartiger Inkontinenzartikel ist beispielsweise bekannt aus DE 10 2007 055 628 A1.

Inkontinenzartikel in Höschenform unterscheiden sich prinzipiell von öffenbaren und schließbaren Inkontinenzartikeln in üblicher Windelform dadurch, dass durch die Höschenform der Hüftumfang schon vorgegeben ist und die Anpassung an unterschiedliche Körpergrößen ausgehend von einer Anzahl von Grundgrößen durch eine elastische Dehnbarkeit des Artikels erreicht wird. Hierfür werden in der Regel Elastifizierungsmittel, insbesondere in Form von Bändern oder Fäden, häufig als Lycra-Fäden bezeichnet, in vorgedehntem Zustand (Stretch-Bond-Verfahren) mit Chassismaterialien des Inkontinenzartikels verbunden, das heißt, sie werden in vorgedehntem Zustand an den Chassismaterialien z.B. mittels Kleber fixiert. Infolge ihrer Vorspannung raffen diese Elastifizierungsmittel die Chassismaterialien zusammen und bilden dabei Fältelungen, die typischerweise quer zur Vorspannungsrichtung der Elastifizierungsmittel, hier also in Längsrichtung verlaufen. Der Inkontinenzartikel bzw. die elastifizierten Chassismaterialien des Inkontinenzartikels lassen sich dann wieder elastisch dehnen, wenn der Inkontinenzartikel wie ein Höschen an den Benutzer angelegt wird. Die Chassismaterialien selbst sind hingegen vorzugsweise undehnbar und lassen sich daher in wohldefinierter Weise in der Förderebene in einem flach oder eben ausgebreiteten Zustand führen, so dass die Elastifizierungsmittel dann mit definierter Vorspannung angefügt werden können.

Zur unlösbaren Verbindung des Bauchabschnitts bzw. des Rückenabschnitts mit dem Schrittabschnitt im jeweiligen Überlappungsbereich kann eine vollflächige Fügung, insbesondere Verklebung, oder eine nicht vollflächige Fügung gewählt werden. Häufig wird zum Fügen großflächiger Bereiche bei vorbekannten Hygieneartikeln ein vollflächiger Kleberauftrag oder ein im Wesentlichen die gesamte Fläche erfassender spiralförmiger, durch Düsen aufgebrachter Kleberauftrag verwendet. Es gibt aber auch Hygieneartikel, bei denen ein anderer Weg beschritten wird; so lehrt beispielsweise US 2004/0116886 A1, keine großflächigen Gebiete des jeweiligen Überlappungsbereichs zu fügen; auf diese Weise soll die Gestalt und Flexibilität der Komponenten im Überlappungsbereich frei bleiben und jedenfalls nicht durch eine Fügeverbindung nachteilig beeinflusst werden. Die gattungsgemäße DE 10 2007 056 126 A1 und EP 2 241 296 A1 sowie die WO 2011/055546 A1 und die EP 2 377 500 A1 erwähnen unter zahlreichen Möglichkeiten auch die Möglichkeit, die Komponenten mittels einer streifenförmigen Kleberstruktur miteinander zu fügen.

US 7,250,549 lehrt, im jeweiligen vorderen bzw. hinteren Überlappungsbereich jeweils Gebiete unterschiedlicher Klebereigenschaften vorzusehen. Dabei soll ein hüftabgewandtes d.h. dem Zentrum des Schrittabschnitts zugewandtes in Querrichtung erstrecktes Gebiet ein größeres Flächengewicht des Klebers aufweisen als ein diesbezüglich hüftseitig liegendes Gebiet. Die Gebiete können vollflächig oder linien-, spiral- oder punktförmig mit Kleber beschichtet sein.

US 7,591,810 B2 lehrt, den Schrittabschnitt und den Bauchabschnitt bzw. den Schrittabschnitt und den Rückenabschnitt nur sehr partiell miteinander zu verbinden.

Wie schon eingangs erwähnt, führt die flächenhafte Elastifizierung des Bauchbands und Rückenbands in Quer- oder Hüftumfangsrichtung zu einer elastischen Raffung der Chassismaterialien, wodurch eine Anpassung des höschenförmigen Inkontinenzartikels an den jeweiligen Körperumfang erfolgt bzw. erst möglich ist. Infolge dieser Raffung bilden sich Fältelungen, die auch bei mehr oder weniger starkem Ausdehnen als in der Längsrichtung des Hygieneartikels erstreckt wahrnehmbar sind. Man erkennt zudem den Verlauf der in Quer- oder Hüftumfangsrichtung erstreckten Elastifizierungsmittel. Im vorderen und hinteren Überlappungsbereich sind diese in Längsrichtung erstreckten Fältelungen aufgrund der versteifenden Wirkung des Schrittabschnitts in geringerem Maße ausgebildet; sie sind dort bei vorbekannten Artikeln jedoch typischerweise ebenfalls wahrnehmbar. Da sich auch im Überlappungsbereich Elastifizierungsmittel in ungefährer Querrichtung erstrecken, da sie typischerweise in der Maschinenrichtung von Bauchabschnitt und Rückenabschnitt, also in Querrichtung, zugeführt werden und dabei bezogen auf den Inkontinenzartikel einen Seitenwechsel vornehmen, ergeben sich weitere Strukturen, die zumindest optisch wahrnehmbar sind. Auch wenn die erwähnten Elastifizierungsmittel im jeweiligen Überlappungsbereich von Schrittabschnitt und Bauchabschnitt bzw. Schrittabschnitt und Rückenabschnitt ihrer elastifizierenden Wirkung benommen sind, d. h. in einer geeigneten Weise deaktiviert, insbesondere geschnitten sind, ist der Verlauf der Elastifizierungsmittel am fertigen Produkt erkennbar. Es bildet sich eine ungefähr in der Querrichtung erstreckte Struktur, die optisch erkennbar ist und eine technische Anmutung des Artikels vermittelt.

Der vorliegenden Erfindung liegt die Aufgabe zugrunde, die Verbindung der drei Komponenten des Hygieneartikels, also die Verbindung von Schrittabschnitt und Bauchabschnitt im vorderen Überlappungsbereich und von Schrittabschnitt und Rückenabschnitt im hinteren Überlappungsbereich, im Hinblick auf die vorstehend genannten Gesichtspunkte zu verbessern. Die Verbindung soll einerseits den im Betrieb des Inkontinenzartikels auftretenden Belastungssituationen sicher Stand halten. Des Weiteren soll auch ein optisch ansprechendes Erscheinungsbild des Inkontinenzartikels im Hinblick auf die vorstehend geschilderten Aspekte erreicht werden.

Diese Aufgabe wird durch einen Inkontinenzartikel mit den Merkmalen des Anspruchs 1 gelöst.

Es wurde erfindungsgemäß festgestellt, dass der erfindungsgemäße Kleberauftrag in Form einer Vielzahl von in Querrichtung erstreckten Klebestreifen im Zusammenwirken mit den zumeist vliesstoffbasierten oder vliesstoffhaltigen Materialien oder Verbundmaterialien des Bauchabschnitts und des Rückenabschnitts und gegebenenfalls auch des Schrittabschnitts geeignet ist, bei der äußeren Sichtseite des Überlappungsbereichs eine visuell und/oder haptisch wahrnehmbare Struktur zu bilden, die mit dem optisch und/oder haptisch erkennbaren Verlauf der Elastifizierungsmittel im jeweiligen Überlappungsbereich harmoniert bzw. übereinstimmt. Auf diese Weise kann der Verlauf der Elastifizierungsmittel im Überlappungsbereich, wo diese Elastifizierungsmittel häufig auch deaktiviert sind, kaschiert werden, derart, dass sie und die kleberbedingte Struktur nicht mehr unterschieden werden können. Dies ist in optischer Hinsicht ansprechend.

Diese im jeweiligen Überlappungsbereich in Querrichtung erstreckte visuell bzw. haptisch wahrnehmbare Struktur wird also durch den streifenförmigen Kleberauftrag gebildet. Dies kommt dadurch zustande, dass streifenförmige unbeleimte Gebiete des Bauchabschnitts und des Rückenabschnitts sich vom Material des Schrittabschnitts abheben können, während die beleimten streifenförmigen Bereiche niedergehalten werden. Dieses Phänomen wird auch dadurch weiter unterstützt, dass die Chassismaterialien von Bauchabschnitt und Rückenabschnitt in der Regel faserige Vliesmaterialien sind, die den Kleber gewissermaßen dreidimensional aufzunehmen vermögen. Dies führt zu einer weiteren Versteifung, welche einerseits der Bildung von in Längsrichtung erstreckten Fältelungen entgegenwirkt und andererseits die Ausbildung einer in Querrichtung erstreckten Struktur begünstigt. Im Ergebnis wird durch diese erfindungsgemäße Strukturbildung eine Kaschierung oder Unkenntlichmachung der im Überlappungsbereich ungefähr in Querrichtung verlaufenden und gegebenenfalls deaktivierten Elastifizierungsmittel erreicht. Jedenfalls fallen diese Elastifizierungsmittel als technische Elemente weniger auf als bei vorbekannten hier in Rede stehenden Inkontinenzartikeln. Auch die generelle Längsfältelung aufgrund der zusammenziehenden Wirkung von in Hüftumfangsrichtung erstreckten Elastifizierungsmitteln im vorderen und hinteren Überlappungsbereich der Chassismaterialien wird reduziert.

Es wurde weiter erfindungsgemäß festgestellt, dass bei einer derartigen unlösbaren Fügung der Komponenten die Menge der hierfür erforderlichen Klebermaterialien reduziert werden kann verglichen mit einem vollflächigen oder beispielsweise spiralförmigen Kleberauftrag ohne dass hiermit Nachteile verbunden sind. Ferner wurde festgestellt, dass bei Verwendung einer Vielzahl von in Querrichtung erstreckten Klebestreifen die typischerweise im Betrieb des Hygieneartikels in Längsrichtung auftretenden Kräfte, die im Überlappungsbereich bzw. über den Überlappungsbereich vom Schrittabschnitt in den Bauchabschnitt bzw. in den Rückenabschnitt eingetragen werden, dann ebenso gut aufgenommen werden können, wenn sich die Vielzahl von Klebestreifen im Wesentlichen über die gesamte Ausdehnung des jeweiligen Überlappungsbereichs erstreckt. Dies bedeutet aber nicht, dass sich der Kleber zwingend bis zum jeweiligen geometrischen Rand des Überlappungsbereichs erstrecken muss; vielmehr ist es bevorzugt, dass der Kleber einen gewissen Abstand in der Größenordnung eines oder weniger Millimeter vom geometrischen Rand des Überlappungsbereichs einhält, um zu vermeiden, dass Kleber aus dem Überlappungsbereich austritt und die Herstellungsvorrichtung verunreinigt oder auch am fertigen Produkt sichtbar wird. Es hat sich weiter als vorteilhaft erwiesen, dass die Vielzahl von Kleberstreifen in der Querrichtung erstreckt ist, und zwar gerade im Hinblick darauf, dass Kräfte eines vollgesogenen Absorptionskörpers schwerkraftbedingt typischerweise in der Längsrichtung, also quer zur Erstreckung der Klebestreifen, wirken. Diese Kräfte können aufgrund der Quererstreckung der Klebestreifen sogar besser und gleichmäßiger über den jeweiligen Überlappungsbereich in die Hüftumfangsrichtung des Bauchabschnitts und des Rückenabschnitts weitergeleitet werden bzw. von diesen aufgenommen werden.

Die Aufbringung der Vielzahl von im Wesentlichen den gesamten jeweiligen Überlappungsbereich erfassenden Klebestreifen erfolgt typischerweise im Zuge eines sogenannten Kontaktauftrags, bevorzugt unter Verwendung von Schablonentechnik. Hierfür können an sich grundsätzlich bekannte Beleimungsvorrichtungen, beispielsweise das System TrueCoat™ der Firma Nordson Deutschland aus Erkrath, Deutschland, verwendet werden. Bei dem System TrueCoat™ handelt es sich um eine Schlitzdüsen-Flächenauftragsvorrichtung, wobei die Schlitzdüsen-Auftragsköpfe konfigurierbar sind und somit einen präzisen kontinuierlichen oder intermittierenden Klebstoffauftrag ermöglichen. Auch die Aufbringung des Klebstoffs unter Verwendung eines Blechs mit streifenförmigen Aussparungen ist möglich. Die Beleimung erfolgt vorteilhafterweise in Maschinenrichtung der den Bauchabschnitt und Rückenabschnitt bildenden Materialbahnen, also mit in Maschinenrichtung erstreckten Klebestreifen, so dass die Vielzahl von Klebestreifen - wie schon mehrfach erwähnt - im fertigen Produkt dann im Wesentlichen quer zur Längsrichtung, also in Quer- oder Hüftumfangsrichtung, verläuft.

Weiter erweist es sich als vorteilhaft, wenn die Beleimung der Bahnen in der Maschinenrichtung getaktet erfolgt, d.h. Kleber tatsächlich nur in den schon erwähnten Überlappungsbereichen zwischen Schrittabschnitt und Bauchabschnitt bzw. zwischen Schrittabschnitt und Rückenabschnitt aufgetragen wird.

Es erweist sich weiter als vorteilhaft, wenn die Breite von Klebestreifen quer zu ihrer Erstreckung wenigstens 1 mm bis höchstens 5 mm, insbesondere bis höchstens 4 mm und weiter insbesondere bis höchstens 3 mm und vorzugsweise 2 mm beträgt.

Die Breite der kleberfreien Streifen beträgt zweckmäßigerweise quer zu ihrer Erstreckung wenigstens 1 mm bis höchstens 15 mm, insbesondere bis höchstens 10 mm, insbesondere bis höchstens 5 mm und weiter insbesondere bis höchstens 3 mm.

Wie bereits erwähnt ist nach der Erfindung in dem jeweiligen Überlappungsbereich, also dem hinteren und/oder dem vorderen Überlappungsbereich, randseitig ein breiterer Klebestreifen vorgesehen. Dies bedeutet, dass in einem in Längsrichtung hüftseitigen Randbereich des Überlappungsbereichs und/oder in einem in Längsrichtung hüftabgewandten Randbereich des Überlappungsbereichs ein in Querrichtung verlaufender breiterer Klebestreifen vorgesehen ist. Dieser hüftseitige Randbereich bzw. hüftabgewandte Randbereich des Überlappungsbereichs erfasst dabei jeweils höchstens 20 %, insbesondere höchstens 18 %, insbesondere höchstens 15 %, insbesondere höchstens 12 %, insbesondere höchstens 10 % der Längserstreckung des jeweiligen vorderen oder hinteren Überlappungsbereichs. In Längsrichtung zwischen diesen Randbereichen sind dann in jedem Fall die Vielzahl von schmalen und in Querrichtung erstreckten Klebestreifen vorgesehen. Die Breite der erwähnten randseitigen Klebestreifen quer zu ihrer Erstreckung beträgt wenigstens 5 mm, insbesondere wenigstens 8 mm, insbesondere wenigstens 12 mm, insbesondere bis höchstens 20 mm, insbesondere bis höchstens 16 mm, insbesondere bis höchstens 14 mm. Sofern in dem Überlappungsbereich nur an einem Rand ein breiterer Klebestreifen vorgesehen ist, so erweist es sich als vorteilhaft, wenn dieser breitere Klebestreifen am hüftfernen Randbereich des Überlappungsbereichs vorgesehen ist, um dort eine stabile Fügung zu erreichen, die ein Einreißen verhindert.

Als besonders vorteilhaft erweist es sich, wenn das Verhältnis der Breite von Klebesteifen zur Breite von unmittelbar benachbarten kleberfreien Streifen 0,2 - 3,0, insbesondere 0,2 - 2,0, insbesondere 0,2 bis 1,5, insbesondere 0,2 - 1,0, insbesondere 0,4 - 0,8, insbesondere 0,5 - 0,7 beträgt. Somit wird in vorteilhafter Weise eine fein und gleichmäßig verteilte Abfolge von Klebestreifen und kleberfreien Streifen bereitgestellt, was eine gleichmäßige Fügebereichsverteilung und damit verbunden eine Fügestabilität im Überlappungsbereich bei gleichmäßiger Verteilung möglicher Versteifung durch die Klebestreifen bereitstellt.

Auch im Hinblick auf eine möglichst gleichmäßige visuell und/oder haptisch erkennbare Gestaltung oder Strukturierung infolge der Vielzahl der Klebestreifen erweist es sich als vorteilhaft, wenn die Breite zumindest derjenigen Klebestreifen, die bezüglich randseitiger Klebestreifen innen, also in Längsrichtung zwischen randseitigen Klebestreifen liegen, jeweils gleich groß ist.

Weiter erweist es sich als vorteilhaft, wenn die Breite der kleberfreien Streifen jeweils gleich groß ist.

Ungeachtet der spezifischen Ausbildung und Anordnung der Vielzahl von Klebestreifen erweist es sich als vorteilhaft, wenn die Gesamtfläche der Klebestreifen bezogen auf die Fläche des Überlappungsbereichs von Schrittabschnitt und Bauchabschnitt bzw. von Schrittabschnitt und Rückenabschnitt 35 - 60 %, insbesondere 40 - 55 % und weiter insbesondere 40 -50 % beträgt.

Des Weiteren erweist es sich als vorteilhaft, wenn das Flächengewicht der Kleberbeschichtung bei den Klebestreifen 2 - 20 g/m$^2$, insbesondere 2 - 15 g/m$^2$, insbesondere 2 - 10 g/m$^2$, insbesondere 5 - 10 g/m$^2$ beträgt, wobei das Flächengewicht bei allen Klebestreifen vorzugsweise gleich ist.

Um eine gleichmäßige Einleitung von Kräften, die im Gebrauch des Hygieneartikels durch im Absorptionskörper aufgenommene Flüssigkeit verursacht werden, in das von dem Bauchabschnitt und dem Rückenabschnitt gebildete in Quer- oder Hüftumfangsrichtung durchgehende Bauch- und Rückenband zu erzielen, erweist es sich als vorteilhaft, wenn die Fläche des Überlappungsbereichs von Schrittabschnitt und Bauchabschnitt bezogen auf die Fläche des Bauchabschnitts wenigstens 12 %, insbesondere 15 - 40 %, insbesondere 15 - 35 %,insbesondere 15 - 30 %, insbesondere 20 - 30 %, insbesondere 22 - 30 % beträgt. Weiter erweist es sich als vorteilhaft, wenn die Fläche des Überlappungsbereichs von Schrittabschnitt und Rückenabschnitt bezogen auf die Fläche des Rückenabschnitts wenigstens 20 %, insbesondere 20 - 45 %, insbesondere 20 - 40 %, insbesondere 22 - 40 %, insbesondere 25 - 40 %, insbesondere 28 - 40 % beträgt.

Nach einer bevorzugten Ausführungsform des erfindungsgemäßen Inkontinenzartikels erweist es sich als vorteilhaft, wenn im Überlappungsbereich von Schrittabschnitt und Bauchabschnitt und/oder im Überlappungsbereich von Schrittabschnitt und Rückenabschnitt zwei äußere randseitige Klebestreifen, also ein hüftferner und ein hüftnaher randseitiger Klebestreifen, und in Längsrichtung zwischen diesen eine Vielzahl von innenliegenden Klebestreifen vorgesehen sind, wobei die Breite der randseitigen Klebestreifen größer als die Breite der innenliegenden Klebestreifen ist, insbesondere wenigstens das 4-fache, insbesondere wenigstens das 5-fache und weiter insbesondere höchstens das 8-fache, insbesondere höchstens das 7-fache der Breite der innenliegenden Klebestreifen beträgt. Es wäre auch denkbar, dass an einem jeweiligen hüftseitigen oder hüftabgewandten Randbereich des jeweiligen Überlappungsbereichs mehrere breitere randseitige Klebestreifen vorgesehen sind, solange der Randbereich die vorausgehend erläuterten Abmessungen in der Längsrichtung nicht überschreitet.

Bei einer bevorzugten Ausführungsform des erfindungsgemäßen Inkontinenzartikels sind im Überlappungsbereich von Bauchabschnitt und Schrittabschnitt wenigstens 8, insbesondere wenigstens 10 Klebestreifen vorgesehen und im Überlappungsbereich von Rückenabschnitt und Schrittabschnitt sind wenigstens 15, insbesondere wenigstens 18 und weiter insbesondere wenigstens 20 Klebestreifen vorgesehen.

Es erweist sich weiter als vorteilhaft, wenn die eingangs erwähnten zweiten Elastifizierungsmittel, die in einem schrittseitigen und den Beinöffnungen zugewandten Bereich des Bauchabschnitts und des Rückenabschnitts vorgesehen sind, entlang ihrer Erstreckung in Richtung auf die Längsmittelachse und in Richtung auf den jeweiligen Überlappungsbereich bogenförmig sich auffächernd mit zunehmendem Abstand voneinander verlaufen. Auf diese Weise kann die in der Fläche des schrittseitigen und den Beinöffnungen zugewandten Bereichs des Bauchabschnitts und des Rückenabschnitts wirkende Rückstellkraft beeinflusst werden. Sie kann in Richtung auf die Längsmittelachse auf diese Weise reduziert werden.

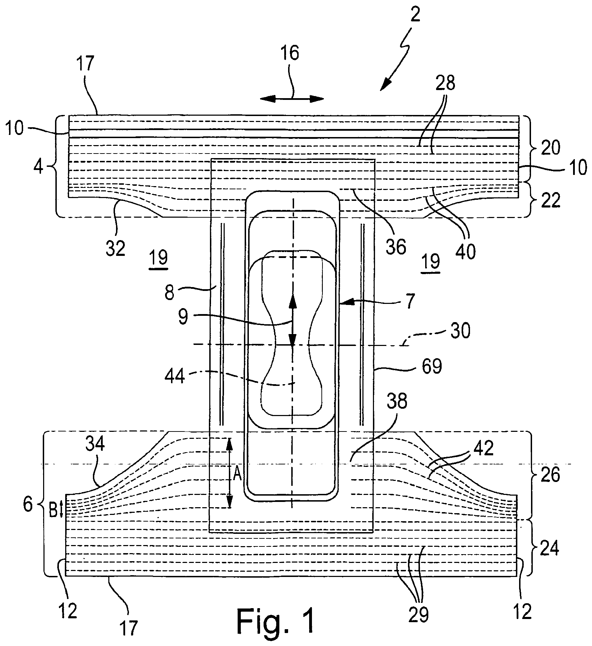

Diese Auffächerung der zweiten Elastifizierungsmittel lässt sich auch quantitativ näher bezeichnen. Beispielsweise haben die in Figur 1 dargestellten zweiten Elastifizierungsmittel des Rückenabschnitts in den Seitennahtbereichen einen minimalen Abstand von 3 bis 8 mm voneinander (Abstand von unmittelbar nebeneinander liegenden Elastifizierungsmitteln) und an einem Absorptionskörperrand oder einem Längsrand des Schrittabschnitts einen maximalen Abstand (Abstand von unmittelbar nebeneinander liegenden Elastifizierungsmitteln) von 7 bis 35 mm voneinander. Es lässt sich ein Auffächerungsgrad F wie folgt definieren:

$$F = (A-B)/B * 100\%.$$

Dieser Auffächerungsgrad kann vorteilhafterweise zwischen 50 und 900 %, insbesondere zwischen 100 und 700 % und weiter insbesondere zwischen 150 bis 550 % liegen. Er ist im Rückenabschnitt vorteilhafterweise größer als im Bauchabschnitt. Dabei sind die Größen A und B definiert als der Abstand des in Längsrichtung äußersten zweiten Elastifizierungsmittels von dem in Längsrichtung innersten zweiten Elastifizierungsmittel (also nicht der Abstand von unmittelbar nebeneinander liegenden Elastifizierungsmitteln), und zwar A als der maximale Abstand, insbesondere am Längsrand des Schrittabschnitts, und B als der minimale Abstand, insbesondere im Seitennahtbereich.

Weiter erweist es sich als vorteilhaft, wenn die ersten und/oder die zweiten Elastifizierungsmittel im Überlappungsbereich von Schrittabschnitt und Bauchabschnitt und im Überlappungsbereich von Schrittabschnitt und Rückenabschnitt parallel zu den Klebestreifen verlaufen. Typischerweise werden die Elastifizierungsmittel derart eingebracht, dass sie sich in Quer- oder Hüftumfangsrichtung, also in der Maschinenrichtung derjenigen den späteren Bauchabschnitt und Rückenabschnitt bildenden Flachmaterialbahnen endlos erstrecken. Sie erstrecken sich daher zumindest zunächst durchgehend über die gesamte Quererstreckung des Inkontinenzartikels. Auf diese Weise kommt es typischerweise zu einem Unterqueren des Absorptionskörpers durch die Elastifizierungsmittel im jeweiligen vorderen bzw. hinteren Überlappungsbereich von Schrittabschnitt und Bauchabschnitt bzw. von Schrittabschnitt und Rückenabschnitt, so dass der Absorptionskörper des Schrittabschnitts oberhalb der Elastifizierungsmittel oder körperzugewandt zu liegen kommt. Solchenfalls erweist es sich als besonders zweckmäßig, wenn die Elastifizierungsmittel zumindest dort, wo sie den Absorptionskörper unterfangen, hinsichtlich ihrer elastifizierenden Wirkung benommen sind; hierfür können sie typischerweise in diesem Gebiet entlang ihrer Quererstreckung insbesondere mehrfach geschnitten oder in sonstiger Weise, z.B. durch Einwirkung von Ultraschall, deelastifiziert werden. Dennoch zeigt es sich, dass der Verlauf sowohl der ursprünglich eingebrachten Elastifizierungsmittel als auch der im jeweiligen Überlappungsbereich deaktivierten bzw. deelastifizierten Elastifizierungsmittel von der Außenseite des Inkontinenzartikels erkennbar ist. Wie schon eingangs erwähnt, kann dies durch Vorsehen der Vielzahl von schmalen Klebestreifen jedoch kaschiert werden, was als besonderer Vorteil der vorliegenden Erfindung anzusehen ist.

Die vorausgehend erwähnte bogenförmige Auffächerung kann vorzugsweise derart gestaltet sein, dass ein Abstand der zweiten Elastifizierungsmittel voneinander in einem jeweiligen Seitennahtbereich 3 bis 8 mm beträgt und weiter innen in Richtung auf die Längsmittelachse zu im Bereich eines Absorptionskörperrands zwischen etwa 7 und 35 mm insbesondere zwischen 12 und 30 mm liegt.

Als erste und/oder zweite Elastifizierungsmittel werden vorzugsweise faden- oder bandförmige Elastifizierungsmittel, wie Gummi- oder Polyetherpolyurethanoder Polyesterpolyurethanfäden, vorzugsweise Elastikfäden wie Lycra®- oder Spandex®-Fäden eingesetzt.

Die ersten Elastifizierungsmittel haben vorzugsweise eine Fadenstärke 400 - 1500 dtex, insbesondere 500 - 1400 dtex, insbesondere von 800 - 1400 dtex, insbesondere von 1000 - 1400 dtex, weiter insbesondere von 1100 - 1400 dtex. Die zweiten Elastifizierungsmittel haben vorzugsweise eine Fadenstärke von 500 - 1000 dtex, insbesondere von 600 - 1000 dtex, weiter insbesondere von 700 - 900 dtex.

Die Fadenstärke der Elastifizierungsmittel wird in der Einheit dtex angegeben (1 dtex = 1g/10000 m). Die Fadenstärke wird entsprechend den Prüfvorschriften BISFA, The International Bureau for the Standardization of man-made Fibres, Test methods for bare elastane yarns, Ausgabe 1998, Kapitel 5: "Determination of linear density" (Bestimmung der linearen Dichte) bestimmt. Die Fadenstärke oder lineare Dichte wird durch Bestimmung der Masse eines Prüfling einer bekannten Fadenlänge von 1000 mm (abgetrennt unter einer Standardvorspannung von 0,1 ± 0,01 mN/tex) nach einer Konditionierung unter Standardbedingungen (23°C ± 2°C, 50% ± 5% relative Luftfeuchtigkeit) im entspannten Zustand bestimmt.

Die Fadenstärke (in dtex) errechnet sich aus dem Quotienten der Masse (in g) durch die Länge des Abschnitts (in m) multipliziert mit dem Faktor 10000.

Es werden hierfür fünf Abschnitte mit jeweils etwa 1300 mm Länge des fadenförmigen oder bandförmigen Elastifizierungsmittels unter geringstmöglicher Spannung von einer Rolle oder Packung abgeschnitten, und zwar in unregelmäßigen Abständen von wenigstens 2 m. Diese fünf Abschnitte werden spannungslos relaxiert und unter den Standardbedingungen für wenigstens vier Stunden ruhen gelassen. Sodann wird ein Prüfling von 1000 mm ± 1 mm Länge von dem jeweiligen 1300 mm langen Abschnitt abgeschnitten, während der betreffende Abschnitt unter einer Vorspannung von 0,1 mN/tex gehalten wird. Diese abgetrennten Prüflinge von 1000 mm Länge werden auf eine Genauigkeit von ± 1 % ihrer erwarteten Masse gewogen. Für jeden Prüfling wird dessen Fadenstärke durch Multiplikation der jeweiligen Masse mit dem Faktor 10000 in dtex erhalten. Aus den fünf Prüflingen wird der arithmetische Mittelwert berechnet, der als Fadenstärke für die hier in Rede stehenden Zwecke verwendet wird.

Die ersten und/oder zweiten Elastifizierungsmittel werden vorzugsweise unter einer Vorspannung von 2,5 - 6,0, an den chassisbildenden Hüllmaterialien des Bauchabschnitts und des Rückenabschnitts fixiert (Strech-bonding-Verfahren). Die Vorspannung ist dabei definiert als der Dehnungsgrad eines gedehnten Elastifizierungsmittels gegenüber dem ungedehnten/relaxierten Ausgangszustand des Elastifizierungsmittels im Zustand des Aufbringens und Fixierens der Elastifizierungsmittel in der Herstellungsmaschine. Der Dehnungsgrad errechnet sich also als Verhältnis der gedehnten Länge L' (=Ausgangslänge L + ΔL) zur Ausgangslänge L, also L'/L. Die ersten Elastifizierungsmittel werden vorzugsweise unter einer Vorspannung von 4,0 - 6,0, insbesondere von 4,5 - 5,5 fixiert, wobei deren Vorspannung vorzugsweise größer ist als diejenige der zweiten Elastifizierungsmittel. Die zweiten Elastifizierungsmittel werden vorzugsweise unter einer Vorspannung von vorzugsweise 2,5 - 5,0, insbesondere von 3,0 - 4,5 fixiert.

Der Schrittabschnitt umfasst vorteilhafterweise ein flüssigkeitsundurchlässiges Backsheet-Material und ein Vlies-Topsheet-Material, zwischen denen der Absorptionskörper angeordnet ist. Hierbei bilden das Backsheet-Material und das Vlies-Topsheet-Material gewissermaßen das Chassis des Schrittabschnitts. In Weiterbildung der Erfindung von besonderer Bedeutung erweist es sich als vorteilhaft, wenn das Backsheet-Material und/oder das Topsheet-Material in Querrichtung einen Überhang über den Absorptionskörper bilden und dieser Überhang - in der Summe beidseits des Absorptionskörpers, also in der Summe links und rechts -wenigstens 25 %, insbesondere 30 - 50 %, weiter insbesondere 35 - 50 %, weiter insbesondere 38 - 48 %, weiter insbesondere 40 - 45 % bezogen auf die größte Breite des Schrittabschnitts (d.h. also bezogen auf die maximale Erstreckung des Schrittabschnitts in Querrichtung) beträgt.

Dieser verhältnismäßig große Überhang von Backsheet-Material und/oder Topsheet-Material beidseits der Absorptionskörpers bedeutet also einen breiten Schrittabschnitt mit einen verhältnismäßig schmalen Absorptionskörper. Hierdurch ist es möglich, entlang der Beinöffnungen erstreckte Beinelastifizierungsmittel in dem Schrittabschnitt vorzusehen, die einen verhältnismäßig großen Abstand zum materialreichen und damit biegesteifen Absorptionskörper haben. Dies resultiert wiederum in einer guten Abdichtbarkeit und Anpassbarkeit der beidseitigen Beinöffnungsränder des Schrittabschnitts. Solchenfalls behindert nämlich der materialreiche und gegenüber dünnen Chassismaterialien verwindungssteife Absorptionskörper die Ausbildung eines flüssigkeitsdichten Beinabschlusses nur wenig; es muss daher zur Ausbildung eines flüssigkeitsdichten Beinabschlusses nicht mit extrem hohen Spannungen gearbeitet werden, was sich wiederum positiv auf den Tragkomfort des Inkontinenzartikels auswirkt.

In noch weitergehender Ausbildung der Erfindung erweist es sich als besonders vorteilhaft, wenn die Beinelastifizierungsmittel in Längsrichtung wenigstens 10 mm, insbesondere wenigstens 20 mm vor den zweiten Elastifizierungsmitteln enden. Es ist insbesondere vorteilhaft, wenn die Beinelastifizierungsmittel in Längsrichtung vor dem Bauchabschnitt und/oder vor dem Rückenabschnitt enden. Die von ihnen ausgeübte Spannung und Rückstellkraft beeinflusst daher nicht die erfindungsgemäß vorgesehenen Spannungsverhältnisse innerhalb des schrittseitigen und den Beinöffnungen zugewandten Bereichs des Bauchabschnitts und des Rückenabschnitts, in dem die sich auffächernden zweiten Elastifizierungsmittel vorgesehen sind.

Als Beinelastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®- oder Spandex®-Fäden eingesetzt. Die Beinelastifizierungsmittel haben vorzugsweise eine Stärke von 300 - 1500 dtex, insbesondere von 500 - 1200 dtex, weiter insbesondere von 500 - 900 dtex.

Die Beinelastifizierungsmittel werden vorzugsweise unter einer Vorspannung von 1,5 - 6,0, insbesondere von 2,5 - 4,5 an den chassisbildenden Hüllmaterialien des Schrittabschnitts fixiert.

Die chassisbildenden Materialien von Bauchabschnitt und/oder Rückenabschnitt umfassen vorzugsweise Vliesstoffmaterialien, wie Spinnvliese, Kardenvliese oder Through Air bonded Kardenvliese. Besonders bevorzugt umfasst das chassisbildende Material von Bauchabschnitt und/oder Rückenabschnitt ein Spinnvliesmaterial. Die für Bauchabschnitt und/oder Rückenabschnitt eingesetzten Vliesstoffmaterialien haben vorteilhafterweise ein Flächengewicht von 10 - 30 g/m², weiter vorzugsweise von 15 - 25 g/m². Besonders bevorzugt umfassen der Bauchabschnitt und der Rückenabschnitt ein Spinnvlies, insbesondere aus Polypropylen, insbesondere mit einem Flächengewicht von 15 - 25 g/m².

Geringe Flächengewichte der chassisbildenden Materialien von Bauchabschnitt und/oder Rückenabschnitt, insbesondere umfassend oder bestehend aus Vliesstoffmaterialien, ermöglichen aufgrund ihrer Flexibilität besonders vorteilhaft die Ausbildung der erfindungsgemäßen visuell und/oder haptisch wahrnehmbaren Strukturen.

Der Schrittabschnitt umfasst vorteilhafterweise ein flüssigkeitsundurchlässiges Backsheet-Material und ein Vlies-Topsheet-Material. Das Backsheet umfasst insbesondere eine Folie, insbesondere eines Flächengewichts von 8 - 20 g/m², insbesondere 8 - 16 g/m², weiter insbesondere 8 - 14 g/m². Insbesondere umfasst das Backsheet eine im Gebrauch flüssigkeitsdichte aber gleichwohl atmungsaktive, also wasserdampfdurchlässige, insbesondere mikroporöse Folie.

Der Absorptionskörper umfasst Körperflüssigkeiten absorbierende Materialien wie natürliche oder synthetische Fasern, insbesondere Zellulosefasern, vorzugsweise in Form von Zellstofffluff. Vorzugsweise umfasst der Absorptionskörper außerdem superabsorbierende Materialien (SAP), insbesondere auf Basis oberflächenvernetzter, teilneutralisierter Polyacrylate.

Der Schrittabschnitt bzw. die Längsränder des Schrittabschnitts, welche die Beinöffnungen begrenzen, können gerade oder auch bogenförmig konturiert ausgebildet sein. Die schrittzugewandten Querränder von Bauchabschnitt und Rückenabschnitt, die ebenfalls die Beinöffnungen begrenzen, sind vorteilhafterweise und vorzugsweise bogenförmig konturiert ausgebildet.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Inkontinenzartikels. In der Zeichnung zeigt:

Figur 1 eine Draufsicht auf einen erfindungsgemäßen Inkontinenzartikel, wobei ein Bauchabschnitt, ein Rückenabschnitt und Schrittabschnitt des Inkontinenzartikels noch nicht zur Bildung einer Höschenform verbunden, sondern in flachgelegtem und eben ausgedehntem Zustand dargestellt sind;

Figur 2a,b schematische Schnittansichten des Schrittabschnitts im Bereich der Quermittellinie bzw. im Überlappungsbereich von Schrittabschnitt und Rückenabschnitt;

Figur 3 eine Figur 1 entsprechende Darstellung, wobei die Fixierung des Schrittabschnitts mit dem Bauchabschnitt und dem Rückenabschnitt mittels Klebestreifen erkennbar ist;

Figur 4 eine vergrößerte ausschnittsweise Darstellung im Bereich des Überlappungsbereichs von Schrittabschnitt und Bauchabschnitt bzw. Schrittabschnitt und Rückenabschnitt des Inkontinenzartikels nach Figur 3;

Figur 5 eine schematische Schnittansicht der wesentlichen Einzelkomponenten der Chassismaterialien entlang der Längsmittelachse des Inkontinenzartikels;

Figur 6 eine schematische Ansicht des fertig konfigurierten Inkontinenzartikels

Figur 7 eine Figur 1 entsprechende Darstellung zur Verdeutlichung von Abmessungen;

Figur 8 eine Figur 1 entsprechende Darstellung zur Verdeutlichung des Aufbaus des Absorptionskörpers und der Faltachsen;

Figur 9 eine schematische Längsschnittansicht des Absorptionskörpers entlang der Längsmittelachse;

Figur 10a,b,c drei schematische Ansichten des Inkontinenzartikels zur Verdeutlichung der Faltung und

Figur 11 eine schematische Ansicht des gefalteten Inkontinenzartikels zur Verdeutlichung der Probennahme bei der Dickenbestimmung.

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 in einer Längsrichtung 9 des Inkontinenzartikels 2 erstreckt ist und mit einem wesentlichen Flächenanteil des Bauchabschnitts 4 einerseits und des Rückenabschnitts 6 andererseits überlappt und im Überlappungsbereich auf noch näher zu beschreibende Weise herstellerseitig unlösbar verbunden ist. Wie aus Figur 1 ersichtlich, führt dies zu einer H-förmigen Grundstruktur des Inkontinenzartikels. Die in Figur 1 dargestellten miteinander gefügten Bestandteile werden dann zur Bildung der Höschenform an jeweiligen seitlichen Längsrandabschnitten 10, 12 des Bauchabschnitts 4 und des Rückenabschnitts 6 ebenfalls herstellerseitig miteinander durch übliche Fügeverfahren verbunden, wodurch beidseits Seitennahtbereiche 14 ausgebildet werden. In diesem herstellerseitig gefertigten höschenförmigen Zustand des Inkontinenzartikels 2 erstrecken sich der Bauchabschnitt 4 und der Rückenabschnitt 6 in einer Quer- oder Hüftumfangsrichtung 16 durchgehend und definieren so mit ihrem Hüftrand 17 eine in Hüftumfangsrichtung geschlossene Hüftöffnung 18; ferner begrenzen sie zusammen mit dem Schrittabschnitt 8 Beinöffnungen 19, durch welche hindurch der Benutzer den Inkontinenzartikel wie ein Höschen anlegt.

Der Bauchabschnitt 4 lässt sich in einen hüftseitigen Bereich 20 und in einen schrittseitigen und den Beinöffnungen 19 zugewandten Bereich 22 unterteilen. Eine entsprechende Unterteilung ist im Rückenabschnitt 6 vorgesehen, und zwar ebenfalls in einen hüftseitigen Bereich 24 und einen schrittseitigen und den Beinöffnungen 19 zugewandten Bereich 26.

In dem hüftseitigen Bereich 20 des Bauchabschnitts 4 und in dem hüftseitigen Bereich 24 des Rückenabschnitts 6 sind erste Elastifizierungsmittel 28, 29 vorgesehen, bei denen es sich insbesondere um fadenförmige Elastifizierungsmittel, wie Lycra-Fäden, handeln kann, die in vorgedehntem Zustand, im sogenannten Stretch-Bond-Verfahren, mit den Flachmaterialien (Chassismaterialien) des Bauchabschnitts 4 und des Rückenabschnitts 6 verbunden sind. Diese ersten Elastifizierungsmittel 28, 29 erstrecken sich in Quer- oder Hüftumfangsrichtung 16 von einem Seitennahtbereich 14 zum anderen.

Der schrittseitige und den Beinöffnungen 19 zugewandte Abschnitt 22 und 26 des Bauchabschnitts 4 bzw. des Rückenabschnitts 6 haben eine von der Quer- oder Hüftumfangsrichtung 16 abweichende in Richtung auf eine Quermittelachse 30 des Schrittabschnitts 8 zulaufende Randkontur 32 bzw. 34. Diese Randkontur 32, 34 ist auch in der Darstellung nach Figur 1 bogenförmig und daher zur Begrenzung der Beinöffnungen 19 geeignet.

Durch diesen Verlauf des schrittseitigen und den Beinöffnungen zugewandten Bereichs 22 bzw. 26 wird auch ein verhältnismäßig großer Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist.

Der jeweilige schrittseitige und den Beinöffnungen 19 zugewandte Bereich 22, 26 des Bauchabschnitts 4 bzw. des Rückenabschnitts 6 ist ebenfalls elastifiziert ausgebildet. Dort sind zweite Elastifizierungsmittel 40 bzw. 42 vorgesehen. Die zweiten Elastifizierungsmittel 40, 42 erstrecken sich jeweils ausgehend von den Seitennahtbereichen 14 in Richtung auf eine Längsmittelachse 44 des Inkontinenzartikels. Wie aus Figur 1 zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt. Die zweiten Elastifizierungmittel 40, 42 unterqueren den Schrittabschnitt 8. Sie können im Bereich unterhalb des Absorptionskörpers 7 deaktiviert, d.h. ihrer elastifizierenden Wirkung benommen sein.

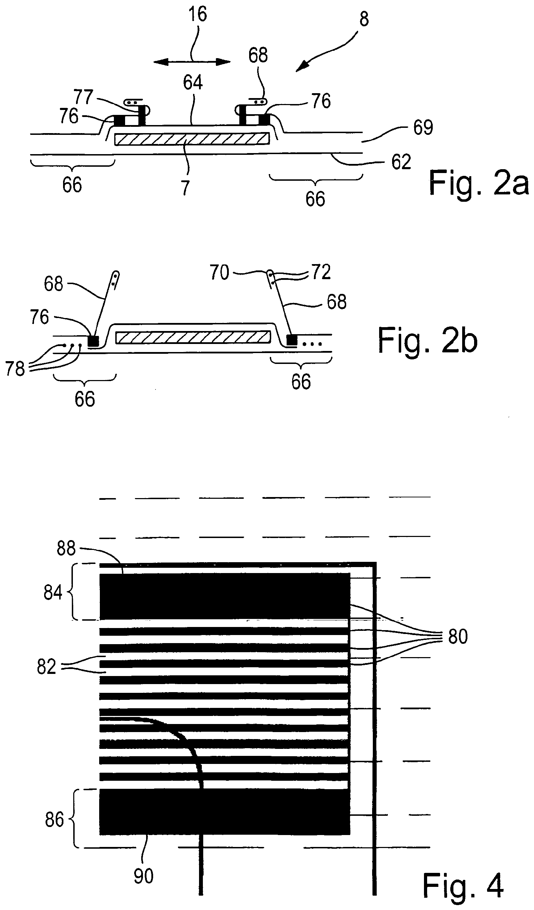

Wie aus Figuren 2a, b ersichtlich umfasst der Schrittabschnitt 8 ein flüssigkeitsundurchlässiges Backsheet-Material 62, das insbesondere von einem atmungsaktiven, jedoch flüssigkeitsdichten Folienmaterial gebildet sein kann, und ein, vorzugsweise auf Vliesbasis beruhendes Topsheet-Material 64. Zwischen dem Backsheet-Material und dem Topsheet-Material ist der Absorptionskörper 7 (nur schematisch dargestellt) angeordnet. Im beispielhaft dargestellten Fall bildet das Backsheet-Material 62 in Querrichtung 16 einen Überhang 66 über den Absorptionskörper 7. Das Topsheet 64 überragt den Absorptionskörper 7 in Querrichtung 16 nur verhältnismäßig geringfügig, und beidseits des Absorptionskörpers 7 ist in Längsrichtung 9 verlaufend jeweils ein aufstehendes Barrieremittel 68 vorgesehen, welches typischerweise als aufstehendes Cuffelement oder Bündchenelement bezeichnet wird und vorzugsweise aus einem hydrophoben, insbesondere flüssigkeitsundurchlässigen Vliesstoffmaterial gebildet ist, welches sich in Querrichtung 16 vorzugsweise bis zu seitlichen Längsrändern 69 des Schrittabschnitts 8 erstreckt. Die distalen Enden 70 der Barrieremittel 68 sind mit weiteren Elastifizierungsmitteln 72 versehen, welche die Barrieremittel 68 im Gebrauch des Inkontinenzartikels gegen die Hautoberfläche des Benutzers anheben. Die seitlichen Barrieremittel 68 sind über schematisch angedeutete Fixierungen 76, 77 auf das Topsheet 64 beziehungsweise auf sich selbst in einer C-förmig gefalteten Konfiguration festgelegt. Außerhalb des Absorptionskörpers 7 also im Bereich des Überhangs 66 sind ferner Beinelastifizierungsmittel 78 vorgesehen, die vorzugsweise mit einem gewissen Abstand vom materialreichen und damit eher biegesteifen Absorptionskörper 7 verlaufen, um einerseits keine zusätzlichen Dehnungs- oder Verwindungskräfte auf den Absorptionskörper auszuüben, was dessen Absorptionsverhalten nachteilig beeinflussen könnte, und um andererseits einen vom Absorptionskörper weitgehend unbeeinflussten flüssigkeitsdichten Beinabschluss zu realisieren. Diese Beinelastifizierungsmittel 78 enden in Längsrichtung 9 mit einem deutlichen Abstand von insbesondere wenigstens 10 mm, vorzugsweise wenigstens 20 mm vor den zweiten Elastifizierungsmitteln 40 und 42 des Bauchabschnitts 4 beziehungsweise des Rückenabschnitts 6. Vorzugsweise enden diese Beinelastifizierungsmittel 78 in Längsrichtung 9 vor dem Bauchabschnitt 4 und dem Rückenabschnitt 6.

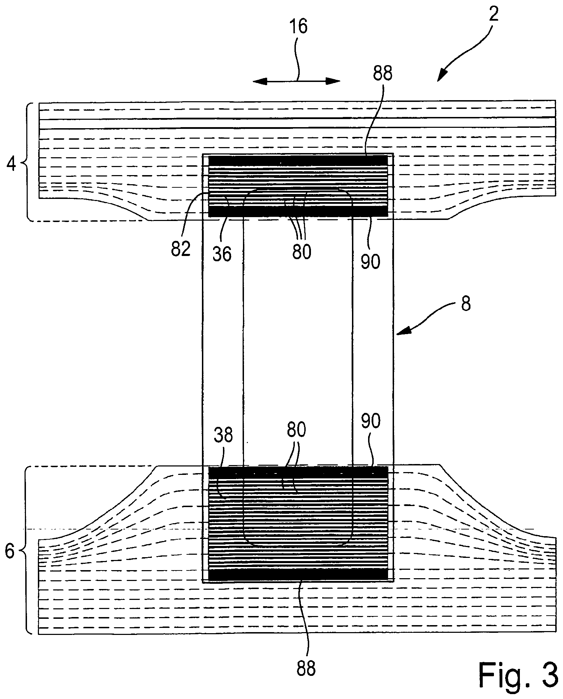

Nachfolgend wird die Fixierung des Schrittabschnitts 8 im vorderen Überlappungsbereich 36 mit dem Bauchabschnitt 4 und im hinteren Überlappungsbereich 38 mit dem Rückenabschnitt 6 beschrieben. Wie anhand der Figuren 3 und 4 ersichtlich, ist hierfür kein vollflächiger Kleberauftrag, sondern eine Vielzahl von im Überlappungsbereich vorgesehener, in Querrichtung 16 erstreckter und parallel zueinander verlaufender Klebestreifen 80 vorgesehen, die durch kleberfreie Streifen 82 voneinander beabstandet sind. Die Klebestreifen 80 erfassen oder überfangen im Wesentlichen den gesamten jeweiligen Überlappungsbereich 36, 38. Im beispielhaft dargestellten, jedoch nicht zwingenden Fall sind in einem in Längsrichtung hüftseitigen Randbereich 84 und in einem in Längsrichtung hüftabgewandten Randbereich 86 des jeweiligen Überlappungsbereichs 36, 38 breitere randseitige Klebestreifen 88 und 90 vorgesehen. Die jeweiligen randseitigen, also hüftzugewandten und hüftfernen Klebestreifen 88, 90 sind mit einer größeren Streifenbreite ausgebildet als die Vielzahl von zwischen ihnen und innen liegenden Klebestreifen 80. Bei einer beispielhaften Ausführungsform beträgt die Breite der randseitigen Klebestreifen 88, 90 quer zu deren Erstreckung 14 mm, die Breite der innen liegenden Klebestreifen 80 2 mm und die Breite der kleberfreien Streifen 82 beträgt 3 mm. Im beispielhaft und bevorzugt dargestellten Fall haben die innen liegenden Klebestreifen 80 vorzugsweise alle dieselbe Breite, und vorzugsweise sind auch die Abstände zwischen ihnen, also die Breite der kleberfreien Streifen 82, gleich. Dessen ungeachtet gelten die eingangs erläuterten Ausführungen zu den Abmessungen und dort beschriebenen Verhältnissen sowie zum Flächengewicht der Kleberbeschichtung bei den Klebestreifen. Auch die Fläche des vorderen und hinteren Überlappungsbereichs 36, 38 bezogen auf die Fläche des Bauchabschnitts 4 bzw. des Rückenabschnitts 6 liegt innerhalb der eingangs erläuterten bevorzugten Grenzen.

Man erkennt des Weiteren aus Figur 3 in Zusammenschau mit Figur 1, dass die zweiten Elastifizierungsmittel 40, 42 im jeweiligen Überlappungsbereich 36, 38 parallel zu den Klebestreifen 80 verlaufen. Auch einige wenige der ersten Elastifizierungsmittel 28 verlaufen im beispielhaft dargestellten Fall im vorderen und hinteren Überlappungsbereich 36, 38 (jedoch auf der körperzugewandten Seite des Schrittabschnitts). Auch die zweiten Elastifizierungsmittel 40, 42 wurden in der Querrichtung 16 durchgehend eingebracht; sie sind im jeweiligen Überlappungsbereich 36, 38 durch eingangs erwähnte Maßnahmen deelastifiziert. Es zeigte sich jedoch, dass die zweiten Elastifizierungsmittel auch im deelastifizierten Zustand - wie eingangs erläutert - erkennbar bleiben. Allerdings werden sie durch die Vielzahl von Klebestreifen 80 kaschiert, und somit wird ihre Erkennbarkeit in erfinderischer Weise reduziert.

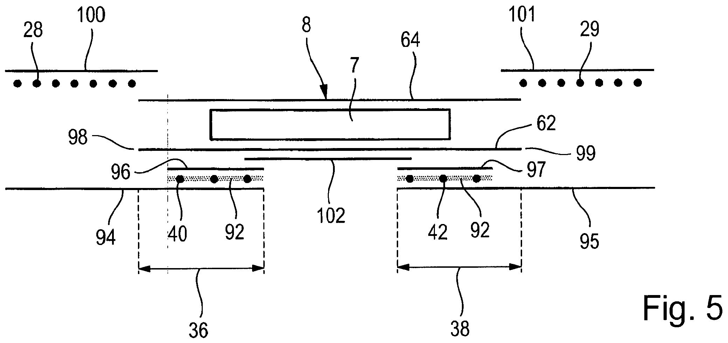

Im bevorzugt dargestellten Fall sind die zweiten Elastifizierungsmittel in einem Leimbett 92 zwischen Chassismateriallagen 94 und 96 bzw. 95 und 97 fixiert (siehe Figur 5). Das Leimbett 92 ist dabei auf eine der Chassismateriallagen 94, 96 bzw. 95, 97 aufgebracht, so dann werden die zweiten Elastifizierungsmittel 40, 42 vorzugsweise endlos auf- bzw. eingelegt und von der weiteren Chassismateriallage überfangen und laminiert. Auf diese Weise werden die zweiten Elastifizierungsmittel 40, 42 fixiert und die Chassismateriallagen 94 und 96 bzw. 95 und 97 flächenhaft durchgehend miteinander gefügt. Bei der körperabgewandten Chassismateriallage 94, 95 handelt es sich um ein atmungsaktives Faservlies, welches der Erstreckung des Bauchabschnitts 4 bzw. Rückenabschnitts 6 entspricht. Bei der Chassismateriallage 96, 97 handelt es sich um ein demgegenüber zurückgesetztes innen liegendes Faservliesmaterial. Es endet in Längsrichtung 9 im bevorzugt dargestellten Fall vor dem Längsende 98, 99 des Schrittabschnitts 8.

Im beispielhaft und bevorzugt dargestellten Fall sind die ersten Elastifizierungsmittel 28, 29 durch Einzelstrangbeleimung zwischen der körperabgewandten Chassismateriallage 94 bzw. 95 und einer weiteren körperzugewandten Chassismateriallage 100, 101 fixiert. Die weitere Chassismateriallage 100, 101 ist wiederum von einem Faservliesmaterial gebildet. Die körperabgewandte und die körperzugewandte Chassismateriallage sind ausschließlich durch die einzelstrangbeleimten ersten Elastifizierungsmittel 28, 29 miteinander verbunden, also nur entlang der Erstreckung dieser ersten Elastifizierungsmittel 28, 29. Die hautfreundlichen Vliesmaterialien sind daher nicht flächenhaft aneinander fixiert, sondern sie können sich voneinander abheben und insbesondere infolge der elastifizierenden Wirkung Fältelungen oder Rüschen bilden. Die körperzugewandte Chassismateriallage 100, 101 erstreckt sich im bevorzugt dargestellten Fall sowohl im Bauchabschnitt 4 als auch im Rückenabschnitt 6 über das jeweilige Längsende 98, 99 des Schrittabschnitts 8 auf dessen körperzugewandter Seite. Es überlappt also diesen Materialübergang und verhindert so eine zu Hautreizungen führende Unstetigkeit.

Man erkennt in Figur 5 des Weiteren, dass das Backsheet 62 des Schrittabschnitts 8 auf seiner körperabgewandten Seite eine Beschichtung 102 aufweist. Diese Beschichtung 102 ist eine Faservliesbeschichtung des im Wesentlichen flüssigkeitsundurchlässigen Backsheets 62. Die Beschichtung 102 erstreckt sich in Längsrichtung 9, jedoch nicht über die gesamte Längserstreckung des Backsheets 62, sondern sie endet verhältnismäßig kurz innerhalb des vorderen und hinteren Überlappungsbereichs 36, 38. Außerhalb des Überlappungsbereichs ist die Beschichtung 102 über die gesamte Erstreckung der körperabgewandten Seite des Backsheets 62 vorgesehen. Die Beschichtung 102 besteht vorzugsweise aus einem Vliesstoff, insbesondere aus einem Spinnvlies, insbesondere aus Polypropylen, insbesondere mit einem Flächengewicht von 10 - 20 $g/m^2$, insbesondere von 12 - 17 $g/m^2$.

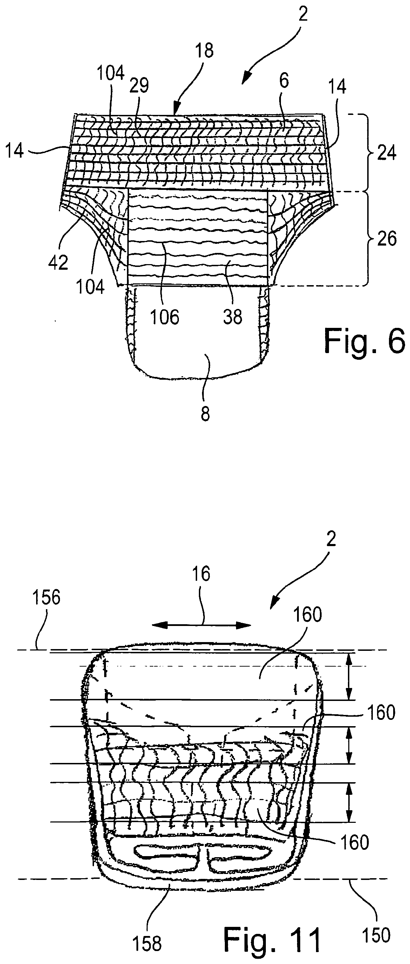

Schließlich zeigt Figur 6 eine schematische Ansicht eines erfindungsgemäßen Inkontinenzartikels im fertig konfigurierten Zustand, in dem der Bauchabschnitt 4 und der Rückenabschnitt 6 durch Ausbildung von Seitennahtbereichen 14 miteinander gefügt sind. Lediglich schematisch angedeutet sind infolge der zusammenziehenden Wirkung der ersten und zweiten Elastifizierungsmittel 28, 29, 40, 42 gebildete Fältelungen oder Rüschen 104, die infolge des Fixierens der Elastifizierungsmittel in vorgedehntem Zustand an den Chassismaterialien (Stretchbondverfahren) gebildet werden. Infolge der Vielzahl von verhältnismäßig feinen Klebestreifen 80 im jeweiligen Überlappungsbereich 36, 38 von Schrittabschnitt 8 und Bauchabschnitt 4 bzw. Rückenabschnitt 6 wird eine visuell und/oder haptisch wahrnehmbare Struktur 106 bei der äußeren Sichtseite des Inkontinenzartikels im jeweiligen Überlappungsbereich 36, 38 gebildet, die hier nur andeutungsweise dargestellt ist. Es wurde erfindungsgemäß festgestellt, dass der streifenförmig aufgebrachte Kleber in die dreidimensional porösen und im Übrigen atmungsaktiv ausgebildeten Faservliesmaterialien, die typischerweise als Chassismaterialien verwendet werden, eindringt und zu einer solchen optisch und/oder haptisch wahrnehmbaren Struktur 106 führt, was sich wie eingangs erläutert als vorteilhaft erweisen kann. Zudem führt die Verbindung von Schrittabschnitt 8 und Bauchabschnitt 4 bzw. Rückenabschnitt 6 durch die Vielzahl von verhältnismäßig schmalen Klebestreifen 80 zu einem sehr wirtschaftlichen Kleberverbrauch, wobei dennoch die erforderlichen Haltekräfte zur sicheren Fügung der drei Komponenten aneinander bereitgestellt werden.

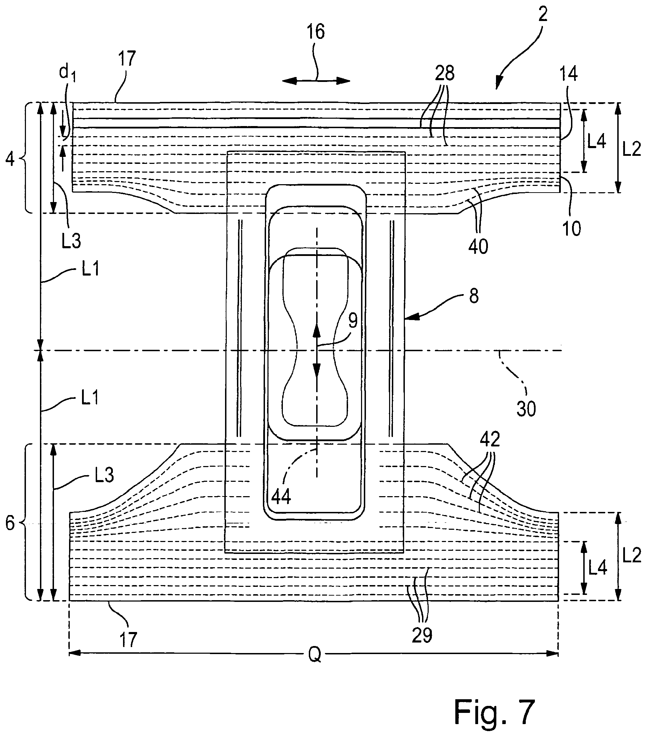

Figur 7 verdeutlicht die Abmessungen, Dimensionen und Verhältnisse eines erfindungsgemäßen Inkontinenzartikels. Man erkennt zunächst, dass die Lage der Quermittelachse 30 die Gesamtlänge des Inkontinenzartikels im flachgelegten Zustand (entsprechend Figur 1) halbiert. Die Quermittelachse 30 bildet auch eine erste in Querrichtung 16 verlaufende Falzachse, um die die Komponenten innerhalb der Herstellungsmaschine gefaltet werden, um die Längsrandabschnitte 10, 12 von Bauchabschnitt 4 und Rückenabschnitt 6 zur Fixierung und Ausbildung von beidseitigen Seitennahtbereichen 14 übereinander zu bringen. Dies findet typischerweise noch unter Führung von endlosen den jeweiligen Bauchabschnitt 4 und Rückenabschnitt 6 bildenden Flachmaterialien, also noch vor der Vereinzelung der Artikel, statt. Man erkennt die Länge L1 zwischen der Quermittelachse 30 und dem jeweiligen Hüftrand 17. Ferner erkennt man die Erstreckung L2 der jeweiligen Seitennaht bzw. des Seitennahtbereichs 14 in Längsrichtung 9, die auch der Länge des jeweiligen Längsrandabschnitts 10 und 12 entspricht. Erfindungsgemäß beträgt das Verhältnis L2/L1 wenigstens 0,42.

Des Weiteren erkennt man den Abstand L4 des äußersten hüftzugewandten ersten Elastifizierungsmittels 28, 29 in Längsrichtung 9 vom innersten schrittzugewandten ersten Elastifizierungsmittel 28, 29. Erfindungsgemäß beträgt das

Verhältnis L4/L1 höchstens 0,3.

Man erkennt des Weiteren, dass die ersten Elastifizierungsmittel 28, 29 einen Abstand $d_1$ voneinander haben, der wenigstens 20 % größer ist als der im Seitennahtbereich 14 bestimmte Abstand der zweiten Elastifizierungsmittel 40, 42 voneinander. Im bevorzugt dargestellten Fall haben die ersten Elastifizierungsmittel 28, 29 alle denselben Abstand $d_1$ voneinander, der wenigstens 10 mm, insbesondere 10 bis 15 mm beträgt. Das Verhältnis d1/L4 beträgt bevorzugt 0,08 bis 0,25.

Des Weiteren erkennt man L3 als die Erstreckung des Bauchabschnitts 4 und Rückenabschnitts 6 in Längsrichtung 9, welche für den Bauchabschnitt 4 insbesondere 135 - 260 mm und für den Rückenabschnitt 6 insbesondere 200 - 320 mm beträgt.

Weiter dargestellt ist die Erstreckung Q des Bauchabschnitts 4 bzw. des Rückenabschnitts 6 in Querrichtung 16, die in Verhältnisse L2/Q oder L4/Q eingeht.

Die ersten Elastifizierungsmittel 28, 29 haben eine um wenigstens 20 % größere Fadenstärke als die zweiten Elastifizierungsmittel 40, 42. Sie sind zudem mit einer um wenigstens 10 % größeren Vorspannung mit den Chassismateriallagen im Bauchabschnitt 4 und im Rückenabschnitt 6 fixiert als die zweiten Elastifizierungsmittel 40, 42.

Auf die weiteren bevorzugten eingangs gegebenen Abmessungen, Dimensionen und Verhältnisse wird verwiesen.

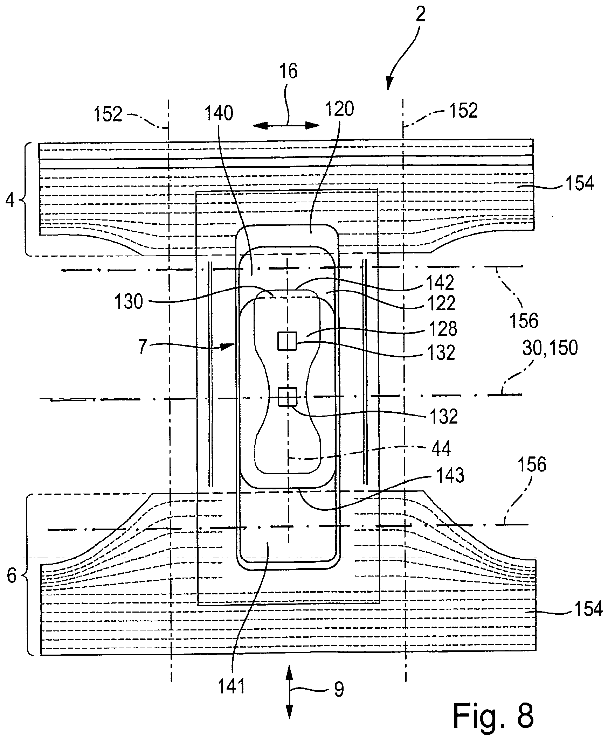

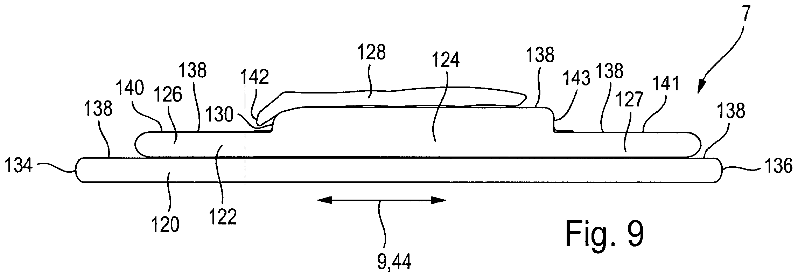

Aus Figuren 8 und 9 ist der Aufbau des Absorptionskörpers 7 in der Draufsicht und in einer Schnittansicht entlang der Längsmittelachse 44 ersichtlich. Der Absorptionskörper 7 umfasst ausgehend von seiner körperabgewandten Seite eine Grundschicht 120 aus zellulosischem Fasermaterial mit einem beispielhaften Flächengewicht von 176 g/m². Je nach exakter flächenhafter Erstreckung enthält die Grundschicht 10 bis 14 g zellulosisches Fasermaterial.

Auf der Grundschicht 120 ist eine Saugkörperschicht 122 abgelegt, die zumindest hinsichtlich des Flächengewichts an Saugkörpermaterial dreidimensional topologisiert ist. Sie weist in einem mittigen Bereich 124 ein höheres Flächengewicht an Saugkörpermaterial auf als in in Längsrichtung 9 vorderen und hinteren Bereichen 126, 127. Das Flächengewicht an zellulosischem Fasermaterial beträgt in dem vorderen und hinteren Bereich 126, 127 der Saugkörperschicht 122 in dem beispielhaft dargestellten Fall 162 g/m² und in dem mittigen Bereich 124 329 g/m². Zusätzlich umfasst die Saugkörperschicht 122 insgesamt etwa 7 g superabsorbierender Polymermaterialien, die homogen gleichförmig in der Saugkörperschicht 122 verteilt angeordnet sind. Die Bereiche 126, 127 und 124 sind in Längsrichtung 9 gegenüber der flächenhaften Erstreckung der Grundschicht 120 zurückgesetzt, wie sich aus Figur 8 ersehen lässt.

Schließlich umfasst der Absorptionskörper 7 eine körperzugewandte im beispielhaft und bevorzugt dargestellten Fall sanduhrförmige Flüssigkeitsaufnahme- und -verteilerschicht 128, die überwiegend auf dem mittleren Bereich 124 der Saugkörperschicht 122 erstreckt ist. Die Flüssigkeitsaufnahme- und -verteilerschicht 128 überragt dabei ein bauchabschnittseitiges Längsende 130 des mittleren Bereichs 124 der Saugkörperschicht 122. Sie umfasst ein Flächengewicht an Fasermaterial, und zwar in Form von intravernetzten Zellulosefasern (curled fiber) von beispielhaft 149 g/m² mit einer Gesamtmasse von entsprechend der beispielhaften Erstreckung ungefähr 2,8 g.

Die Grundschicht 120, die drei Bereiche 124, 126 und 127 der Saugkörperschicht 122 und die körperzugewandte Flüssigkeitsaufnahme- und -verteilerschicht 128 haben über ihre flächenhafte Erstreckung gleichförmiges Flächengewicht an Saugkörpermaterialien.

Das Flächengewicht wird messtechnisch wie eingangs beschrieben ermittelt, indem ein Prüfling von 25 mm x 25 mm betrachtet wird, der durch sämtliche soeben beschriebene Schichten des Absorptionskörpers 7 ausgestanzt wird. Der auszustanzende Flächenbereich 132 (25 mm x 25 mm) wird immer bezüglich der Längsmittelachse 44 zentriert, so wie dies in Figur 8 angedeutet ist. Wenn das Flächengewicht in Längsrichtung 9 weiter vorn oder weiter hinten bestimmt wird, wird der Prüfling in entsprechender Weise bezüglich der Längsmittelachse 44 zentriert.

Man erkennt, dass das Flächengewicht an Saugkörpermaterial so ausgehend von der Quermittelachse 30 entlang der Längsmittelachse 44 in Richtung auf ein bauchabschnittseitige Ende 134 und in Richtung auf ein rückenabschnittseitige Ende 136 des Absorptionskörpers 7 stufenförmig abnimmt. Auf diese Weise werden zwischen den Stufen Plateaus 138 gebildet. Im Bereich dieser Plateaus 138 ist das Flächengewicht an Saugkörpermaterial der darunter liegenden Schichten des Absorptionskörpers 7 vorzugsweise aber nicht notwendigerweise konstant.

Bei der dargestellten bevorzugten Ausführungsform des Inkontinenzartikels ist das Flächengewicht des Absorptionskörpers 7 ausgehend von der Quermittelachse 30 nach vorn und nach hinten im Bereich der Überlappung der körperzugewandten Flüssigkeitsaufnahme- und

-verteilerschicht 128 mit dem mittigen Bereich 124 der Saugkörperschicht 122 im Wesentlichen konstant.

Man erkennt in Figuren 8 und 9 Plateaus 140, 141, die sich an eine Abstufung 142, 143 in Längsrichtung 9 nach vorn bzw. hinten anschließen. Im Bereich dieser Plateaus 140, 141 ist das Flächengewicht des Absorptionskörpers 7 gegenüber dem Flächengewicht im Bereich der Quermittelachse 30 wesentlich reduziert.

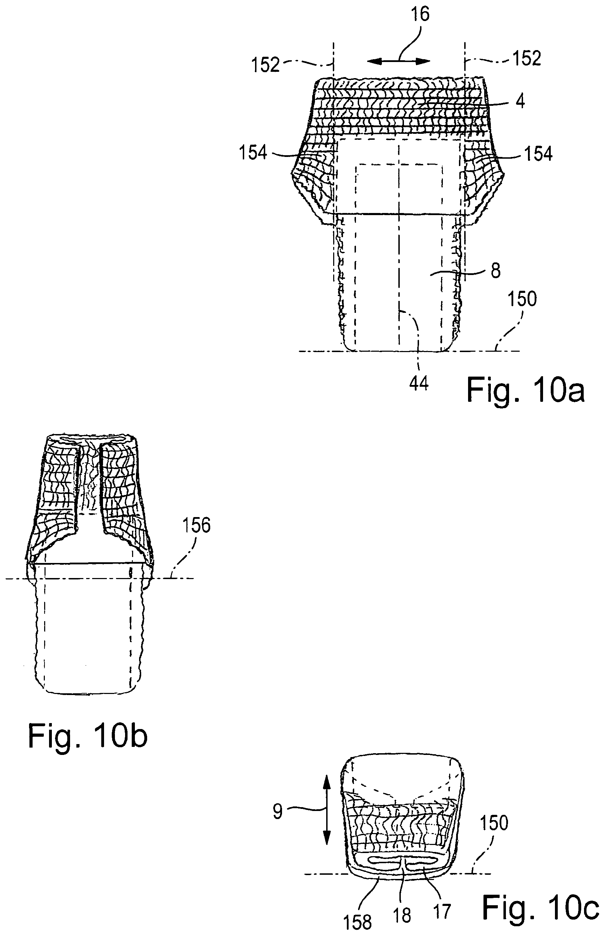

Es wird nun anhand der Figuren 8, 10 und 11 die Faltung des höschenförmigen Inkontinenzartikels für die stapelförmige Anordnung einer Vielzahl von Inkontinenzartikeln in einer Verpackung für die Abgabe in den Handel beschrieben: Wie bereits erwähnt bildet die Quermittelachse 30 eine erste Falzachse 150, um die der Inkontinenzartikel gefaltet wird, so dass Bauchabschnitt 4 und Rückenabschnitt 6 zur Ausbildung von Seitennahtbereichen 14 dauerhaft miteinander gefügt werden können, und zwar durch an sich übliche Fügeverfahren, wie Kleben, Ultraschall, etc.. Des Weiteren sind ungefähr in Längsrichtung 9 erstreckte zweite Falzlinien 152 in Figur 8 lediglich ungefähr angedeutet, da ja die Einfaltung nicht in dem in Figur 8 dargestellten ausgedehnten Zustand erfolgt, sondern nach Fertigstellung des höschenförmigen Inkontinenzartikels in dem in Figur 10a nur schematisch skizzierten Zustand. Ausgehend von diesem in Figur 10a skizzierten Zustand werden beidseitig in Querrichtung 16 seitlich über den Schrittabschnitt 8 überstehende Bereiche 154 des Bauchabschnitts 4 und Rückenabschnitts 6 in Richtung auf die Längsmittelachse 44, und zwar vorzugsweise auf die Außenseite des Bauchabschnitts 4, eingeschlagen, so dass die in Figur 10b ungefähr skizzierte Konfiguration erhalten wird.

Schließlich zeigen die Figuren 8 und 10 eine dritte in Querrichtung 16 verlaufende Falzachse 156, deren Lage bezüglich des Absorptionskörpers 7 aus Figur 8 hervorgeht. Bei weiterer Faltung um diese einzige weitere in Querrichtung 16 erstreckte Falzachse 156 wird die in Figur 10c dargestellte kompakt gefaltete Konfiguration des höschenförmigen Inkontinenzartikels erhalten. Man erkennt, dass der die Hüftöffnung 18 begrenzende Hüftrand 17 in Längsrichtung 9 nicht über die durch die erste Falzachse 150 gebildete äußere Falzkante 158 des Inkontinenzartikels übersteht.

Schließlich verdeutlicht Figur 11, an welchen Stellen die Dicke des insgesamt in die Konfiguration der Figur 10c gefalteten Inkontinenzartikels 2 bestimmt wird. Wie bereits erwähnt, wird der gesamte so gefaltete Inkontinenzartikel 2 über die gesamte Querrichtung 16 mit einem Stanzmesser im Abstand von etwa 10 mm von den Falzkanten bzw. Falzachsen 150 und 156 ausgestanzt, so dass streifenförmige Prüflinge 160 gebildet werden. Anhand dieser sämtliche Lagen des Inkontinenzartikels erfassenden Prüflinge 160 wird dann die Dicke wie eingangs beschrieben ermittelt.

**Patentansprüche**

1. Inkontinenzartikel (2) in Höschenform für die Aufnahme von Körperausscheidungen mit einem vorderen Bauchabschnitt (4) und einem hinteren Rückenabschnitt (6) mit vliesstoffbasierten oder vliesstoffhaltigen Materialien oder Verbundmaterialien, die von separaten und in einer Längsrichtung (9) entlang einer Längsmittelachse (44) voneinander beabstandeten Komponenten gebildet sind, jedoch zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), der sich in der Längsrichtung (9) zwischen Bauchabschnitt (4) und Rückenabschnitt (6) erstreckt und an den Bauchabschnitt (4) und an den Rückenabschnitt (6) in einem jeweiligen Überlappungsbereich (36, 38) unlösbar angefügt ist, wobei der Bauchabschnitt (4), der Rückenabschnitt (6) und der Schrittabschnitt (8) gemeinsam Beinöffnungen (19) des Inkontinenzartikels begrenzen, wobei in dem Bauchabschnitt (4) und dem Rückenabschnitt (6) erste Elastifizierungsmittel (28, 29) vorgesehen sind, die sich in einem Abstand voneinander und parallel zueinander in Quer- oder Hüftumfangsrichtung (16) erstrecken und so den Bauchabschnitt (4) und den Rückenabschnitt (6) flächenhaft elastifizieren, wobei in einem schrittseitigen und den Beinöffnungen (19) zugewandten Bereich (22, 26) des Bauchabschnitts (4) und des Rückenabschnitts (6) zweite Elastifizierungsmittel (40, 42) vorgesehen sind, die sich insbesondere ausgehend von den beiden Seitennahtbereichen (14) in Richtung auf eine Längsmittelachse (44) des Inkontinenzartikels erstrecken und sich dabei bis in den Überlappungsbereich (36,38) von Schrittabschnitt (8) und Bauchabschnitt (4) bzw. von Schrittabschnitt (8) und Rückenabschnitt (6) erstrecken, wobei sie dort ihrer elastifizierenden Wirkung benommen sein können, insbesondere geschnitten sein können, wobei der Schrittabschnitt (8) mittels einer Vielzahl von im Überlappungsbereich (36) von Schrittabschnitt (8) und Bauchabschnitt (4) und im Überlappungsbereich (38) von Schrittabschnitt (8) und Rückenabschnitt (6) vorgesehener, in Querrichtung (16) erstreckter, parallel zueinander verlaufender und voneinander durch kleberfreie Streifen (82) beabstandeter Klebestreifen (80) unlösbar mit dem Bauchabschnitt (4) und mit dem Rückenabschnitt (6) verbunden ist, und wobei hierdurch entsprechend dem Verlauf der Klebestreifen (80) und der kleberfreien Streifen (82) in Querrichtung (16) erstreckte visuell und/oder haptisch wahrnehmbare Strukturen (106) bei der äußeren Sichtseite des Inkontinenzartikels im Überlappungsbereich (36) von Schrittabschnitt (8) und Bauchabschnitt (4) und im Überlappungsbereich (38) von Schrittabschnitt (8) und Rückenabschnitt (6) gebildet werden und wobei in einem in Längsrichtung (9) hüftseitigen Randbereich (84) des Überlappungsbereichs (36, 38) und/oder in einem in Längsrichtung (9) hüftabgewandten Randbereich (86) des Überlappungsbereichs (36, 38) ein in Querrichtung (16) verlaufender breiterer Klebestreifen (88, 90) vorgesehen ist, wobei der jeweilige hüftseitige Randbereich (84) und der hüftabgewandte Randbereich (88) des Überlappungsbereichs (36, 38) jeweils höchstens 20 % der Längserstreckung des jeweiligen Überlappungsbereichs (36, 38) erfasst.

2. Inkontinenzartikel nach Anspruch 1, **dadurch gekennzeichnet, dass** die Breite von Klebestreifen (80) quer zu ihrer Erstreckung wenigstens 1 mm bis höchstens 5 mm, weiter insbesondere bis höchstens 4 mm, weiter insbesondere bis höchstens 3 mm und vorzugsweise 2 mm beträgt.

3. Inkontinenzartikel nach Anspruch 1 oder 2, **dadurch gekennzeichnet, dass** die Breite der kleberfreien Streifen (82) quer zu ihrer Erstreckung wenigstens 1 mm bis höchstens 15 mm, insbesondere bis höchstens 10 mm, insbesondere bis höchstens 5 mm und weiter insbesondere bis höchstens 3 mm beträgt.

4. Inkontinenzartikel nach Anspruch 1, 2 oder 3, **dadurch gekennzeichnet, dass** der jeweilige hüftseitige Randbereich (84) und der hüftabgewandte Randbereich (88) des Überlappungsbereichs (36, 38) jeweils höchstens 18 %, insbesondere höchstens 15 %, insbesondere höchstens 12 %, insbesondere höchstens 10 % der Längserstreckung des jeweiligen Überlappungsbereichs (36, 38) erfasst.

5. Inkontinenzartikel nach einem der vorstehenden Ansprüche, **dadurch gekennzeichnet, dass** die Breite von randseitigen Klebestreifen (88, 90) quer zu ihrer Erstreckung wenigstens 5 mm, insbesondere wenigstens 8 mm, insbesondere wenigstens 12 mm, insbesondere bis höchstens 20 mm, insbesondere bis höchstens 16 mm, insbesondere bis höchstens 14 mm beträgt.

6. Inkontinenzartikel nach einem oder mehreren der vorstehenden Ansprüche, **dadurch gekennzeichnet, dass** das Verhältnis der Breite von Klebesteifen (80) zur Breite von unmittelbar benachbarten kleberfreien Streifen (82) 0,2 - 3,0, insbesondere 0,2 - 2,0, insbesondere 0,2 bis 1,5, insbesondere 0,2 - 1,0, insbesondere 0,4 - 0,8, insbesondere 0,5 - 0,7 beträgt.

7. Inkontinenzartikel nach einem oder mehreren der vorstehenden Ansprüche, **dadurch gekennzeichnet, dass** die Breite zumindest derjenigen Klebestreifen (80), die bezüglich randseitiger Klebestreifen (88, 90) innen liegen, jeweils gleich groß ist.

8. Inkontinenzartikel nach einem oder mehreren der vorstehenden Ansprüche, **dadurch gekennzeichnet, dass** die Breite der kleberfreien Streifen (82) jeweils gleich groß ist.

9. Inkontinenzartikel nach einem oder mehreren der vorstehenden Ansprüche, **dadurch gekennzeichnet, dass** die Gesamtfläche der Klebestreifen (80, 88, 90) bezogen auf die Fläche des Überlappungsbereichs (36, 38) von Schrittabschnitt (8) und Bauchabschnitt (4) bzw. von Schrittabschnitt (8) und Rückenabschnitt (6) 35 - 60 %, insbesondere 40 - 55 % und weiter insbesondere 40 - 50 % beträgt.

10. Inkontinenzartikel nach einem oder mehreren der vorstehenden Ansprüche, **dadurch gekennzeichnet, dass** das Flächengewicht der Kleberbeschichtung bei den Klebestreifen (80, 88, 90) 2 - 20 g/m$^2$, insbesondere 2 -15 g/m$^2$, insbesondere 2 - 10 g/m$^2$, insbesondere 5 - 10 g/m$^2$ beträgt, wobei das Flächengewicht bei allen Klebestreifen vorzugsweise gleich ist.

11. Inkontinenzartikel nach einem oder mehreren der vorstehenden Ansprüche, **dadurch gekennzeichnet, dass** die Fläche des Überlappungsbereichs (36) von Schrittabschnitt (8) und Bauchabschnitt (4) bezogen auf die Fläche des Bauchabschnitts wenigstens 12 %, insbesondere 15 - 40 %, insbesondere 15 - 35 %, insbesondere 15 - 30 %, insbesondere 20 - 30 %, insbesondere 22 - 30 % beträgt.

12. Inkontinenzartikel nach einem oder mehreren der vorstehenden Ansprüche, **dadurch gekennzeichnet, dass** die Fläche des Überlappungsbereichs (38) von Schrittabschnitt (8) und Rückenabschnitt (6) bezogen auf die Fläche des Rückenabschnitts (6) wenigstens 20 %, insbesondere 20 -45 %, insbesondere 20 - 40 %, insbesondere 22 - 40 %, insbesondere 25 - 40 %, insbesondere 28 - 40 % beträgt.

13. Inkontinenzartikel nach einem oder mehreren der vorstehenden Ansprüche, **dadurch gekennzeichnet, dass** im Überlappungsbereich (36) von Schrittabschnitt (8) und Bauchabschnitt (4) und/oder im Überlappungsbereich (38) von Schrittabschnitt (8) und Rückenabschnitt (6) zwei äußere randseitige Klebestreifen (88, 90) und in Längsrichtung (9) zwischen diesen eine Vielzahl von innenliegenden Klebestreifen (80) vorgesehen sind, wobei die Breite der randseitigen Klebestreifen (88, 90) größer als die Breite der innenliegenden Klebestreifen (80) ist, insbesondere wenigstens das 4-fache, insbesondere wenigstens das 5-fache und weiter insbesondere höchstens das 8-fache, insbesondere höchstens das 7-fache der Breite der innenliegenden Klebestreifen beträgt.

14. Inkontinenzartikel nach einem oder mehreren der vorstehenden Ansprüche, **dadurch gekennzeichnet, dass** im Überlappungsbereich (36) von Bauchabschnitt (4) und Schrittabschnitt (8) wenigstens 8, insbesondere wenigstens 10 Klebestreifen (80, 88, 90) vorgesehen sind und im Überlappungsbereich (38) von Rückenabschnitt (6) und Schrittabschnitt (8) wenigstens 15, insbesondere wenigstens 18 und weiter insbesondere wenigstens 20 Klebestreifen (80, 88, 90) vorgesehen sind.

15. Inkontinenzartikel nach einem oder mehreren der vorstehenden Ansprüche, **dadurch gekennzeichnet, dass** die zweiten Elastifizierungsmittel (40, 42) entlang ihrer Erstreckung in Richtung auf die Längsmittelachse (44) des Inkontinenzartikels bogenförmig sich auffächernd mit zunehmendem Abstand voneinander verlaufen.

16. Inkontinenzartikel nach einem oder mehreren der vorstehenden Ansprüche, **dadurch gekennzeichnet, dass** die ersten und/oder die zweiten Elastifizierungsmittel (28, 29; 40, 42) im Überlappungsbereich (36) von Schrittabschnitt (8) und Bauchabschnitt (4) und im Überlappungsbereich (38) von Schrittabschnitt (8) und Rückenabschnitt (6) parallel zu den Klebestreifen (80) verlaufen.

## Claims

1. Incontinence article (2) in pant form for absorbing bodily excretions, with a front stomach section (4) and a rear back section (6) with nonwoven based or nonwoven containing materials or compound materials, which front stomach section (4) and rear back section (6) are formed by separate components which are spaced apart in a longitudinal direction along a longitudinal center axis, which however are connected to each other by the manufacturer at lateral seam regions on both sides to form a stomach and back band which is continuous in transverse or waist circumferential direction (16) having a waist opening (18) that is closed in waist circumferential direction, and with a crotch section (8) having an absorption body (7) and extending in the longitudinal direction (9) between stomach section (4) and back section (6) and is non detachably joined to the stomach section (4) and the back section (6) in a respective overlapping region (36, 38), wherein the stomach section (6), the back section (6) and the crotch section (8) together delimit leg openings (19) of the incontinence article, wherein in the stomach section (4) and the back section (6) first elastifying means (28, 29) are provided which extend spaced apart and parallel to each other in transverse or waist circumferential direction (16) and in this way provide the stomach section (4) and the back section (6) with planar elasticity, wherein in a crotch side region (22, 26) of the stomach section (4) and the back section (6) which crotch side region faces the leg openings (19) second elastifying means (40, 42) are provided which in particular starting from the two lateral seam regions (14) extend in the direction toward a longitudinal center axis (44) of the incontinence article and as far as into the overlapping region (36, 38) of the crotch section (8) and stomach section (4) or crotch section (8) and back section (6) respectively, wherein they may no longer have their elastifying effect at this location and can in particular be cut, wherein the crotch section (8) is non detachably connected with the stomach section (4) and with the back section (6) by multiple adhesive strips (80) which are provided in the overlapping region (36) of crotch section (8) and stomach section (4) and in the overlapping region (38) of crotch section (8) and back section (6), and which extend parallel to each other in transverse direction (16) and are spaced apart by adhesive free strips (82), and wherein thereby visually and tactilely perceptible structures (106) are formed at the outer visual side of the incontinence article in the overlapping region (36) of crotch section (8) and stomach section (4) and in the overlapping region (38) of crotch section and back section (6), which structures correspond to the course of the adhesive strips (80) and the adhesive free strips (82) and extend in transverse direction (16) and wherein in a in longitudinal direction (9) waist-side border region (84) of the overlapping region (36, 38) and/or in a in longitudinal direction waist-averted border region (86) of the overlapping region (36, 38) a wider adhesive strip (88, 90) which extends in transverse direction (16) is provided, and wherein the respective waist-side border region (84) and the waist-averted border region (86) of the overlapping region (36, 38) includes at most 20% of the longitudinal extent of the respective overlapping region (36, 38).

2. Incontinence article according to claim 1, **characterized in that** the width of adhesive strips (80) transverse to their extent is at least 1 mm to at most 5 mm, further in particular to at most 4 mm, further in particular to at least 3 mm and preferably 2 mm.

3. Incontinence article according to claim 1 or 2, **characterized in that** the width of the adhesive-free strips (82) transverse to their extent is at least 1 mm to at most 15 mm, in particular to at most 10 mm, in particular to at most 5 mm and further in particular at most 3 mm.

4. Incontinence article according to claim 1, 2 or 3, **characterized in that** the respective waist-side border region (84) and the waist-averted border region (86) of the overlapping region (36, 38) respectively includes at most 18%, in particular at most 15%, in particular at most 12%, in particular at most 10% of the longitudinal extent of the respective overlapping region (36, 38).

5. Incontinence article according to one opf the preceding claims, **characterized in that** the width of border-side adhesive strips (88, 90) transverse to their extent is at least 5 mm, in particular at least 8 mm, in particular at least 12 mm, in particular to at most 20 mm, in particular to at most 16 mm, in particular at most 14 mm.

6. Incontinence article according to one or more of the preceding claims, **characterized in that** the ratio between the width of adhesive strips (80) to the width of immediately neighboring adhesive-free strips (82) is 0.2-3.0, in particular 0.2 - 2.0, in particular 0.2 to 1.5, in particular 0.2 - 1.0, in particular 0.4 - 0.8, in particular 0.5 - 0.7.

7. Incontinence article according to one or more of the preceding claims, **characterized in that** the width at least of those adhesive strips (80) which are positioned inwardly relative to border-side adhesive strips (88, 90) are of the same size.

8. Incontinence article according to one or more of the preceding claims, **characterized in that** the width of the adhesive-free strips (82) is respectively the same.

9. Incontinence article according to one or more of the preceding claims, **characterized in that** the total surface of the adhesive strips (80, 88, 90) is 35%-60%, in particular 40-55% and further 40 - 50% of the surface of the overlapping region (36, 38) of crotch section (8) and stomach section (4) or of crotch section (8) and back section (6).

10. Incontinence article according to one or more of the preceding claims, **characterized in that** the mass per area of the glue coating in the adhesive strips (80, 88, 90) is 2 - 20 g/m$^2$, in particular 2 - 15 g/m$^2$, in particular 2 - 10 g/m$^2$, in particular 5 - 10 g/m$^2$, wherein the mass per area in all adhesive strips is preferably the same.

11. Incontinence article according to one or more of the preceding claims, **characterized in that** the surface of the overlapping region (36) of crotch section (8) and stomach section (4) is at least 12%, in particular 15-40%, in particular 15 - 35 %, in particular 15 - 30%, in particular 20 - 30%, in particular 22 - 30% of the surface of the stomach section.

12. Incontinence article according to one or more of the preceding claims, **characterized in that** the surface of the overlapping region (38) of crotch section (8) and back section (6) is at least 20%, in particular 20-45%, in particular 20-40%, in particular 22-40%, in particular 25 - 40%, in particular 28-40% of the surface of the back section (6).

13. Incontinence article according to one or more of the preceding claims, **characterized in that** in the overlapping region (36) of crotch section (8) and stomach section (4) and/or in the overlapping region (38) of crotch section (8) and back section (6) two outer border-side adhesive strips (88, 90) and in longitudinal direction (9) between these, plural inwardly located adhesive strips (80) are provided, wherein the width of the border-side adhesive strips (88, 90) is greater than the width of the inwardly located adhesive strips (80), in particular 4 times, in particular at least 5 times, and further in particular at most 8 times, in particular 7 times the width of the inwardly located adhesive strips.

14. Incontinence article according to one or more of the preceding claims, **characterized in that** in the overlapping region (36) of stomach section (4) and crotch section (8) at least 8, in particular at least 10 adhesive strips (80, 88, 90) are provided and in the overlapping region (38) of back section (6) and crotch section (8) at least 15 in particular at least 18 and further in particular at least 20 adhesive strips (80, 88, 90) are provided.

15. Incontinence article according to one or more of the preceding claims, **characterized in that** the second elastifying means (40, 42) fan out arch-shaped with increasing distance to one another along their extent in the direction toward the longitudinal center axis (44) of the incontinence article.

16. Incontinence article according to one or more of the preceding claims, **characterized in that** the first and/or the second elastifying means (28, 29; 40, 42) in the overlapping region (36) of crotch section (8) and stomach section (4) and in the overlapping region (38) of crotch section (8) and back section (6) extend parallel to the adhesive strips.

## Revendications

1. Article d'incontinence (2) sous forme de culotte, destiné à recueillir les excrétions corporelles, comprenant une portion ventrale avant (4) et une portion dorsale arrière (6) ayant des matériaux ou matériaux composites sur la base de non-tissé ou contenant du non-tissé qui sont formées par des composants séparés et espacés l'un de l'autre dans une direction longitudinale (9) suivant un axe médian longitudinal (44), mais qui, pour la formation d'une bande ventrale et dorsale continue dans le sens transversal ou dans le sens du tour de hanches (16), avec une ouverture de hanche (18) fermée dans le sens du tour de hanches, sont reliées entre elles par le fabricant au niveau de zones de couture latérale (14) situées de part et d'autre, et comprenant une portion d'entrejambe (8) présentant un corps absorbant (7), qui s'étend dans la direction longitudinale (9) entre la portion ventrale (4) et la portion dorsale (6) et qui est rapportée de manière inamovible à la portion ventrale (4) et à la portion dorsale (6) dans une zone de chevauchement (36, 38) respective, dans lequel ladite portion ventrale (4), ladite portion dorsale (6) et ladite portion d'entrejambe (8) délimitent ensemble des ouvertures de jambe (19) de l'article d'incontinence, dans lequel de premiers moyens d'élastification (28, 29) sont prévus dans la portion ventrale (4) et dans la portion dorsale (6), qui s'étendent à distance les uns des autres et parallèlement entre eux dans le sens transversal ou dans le sens du tour de hanches (16) et qui élastifient ainsi en nappe lesdites portions ventrale (4) et dorsale (6), dans lequel de seconds moyens d'élastification (40, 42) sont prévus dans une zone (22, 26) de la portion ventrale (4) et de la portion dorsale (6), qui est située côté entrejambe et montre vers les ouvertures de jambe (19), seconds moyens d'élastification qui s'étendent en particulier à partir des deux zones de couture latérale (14) en direction d'un axe médian longitudinal (44) de l'article d'incontinence, tout en s'étendant jusque dans la zone de chevauchement (36, 38) des portions d'entrejambe (8) et ventrale (4) ou bien des portions d'entrejambe (8) et dorsale (6), ils y pouvant être dépourvus de leur effet élastifiant, en particulier être coupés, dans lequel ladite portion d'entrejambe (8) est reliée de manière inamovible à la portion ventrale (4) et à la portion dorsale (6) par l'intermédiaire d'une pluralité de rubans adhésifs (80) qui sont prévus dans la zone de chevauchement (36) des portions d'entrejambe (8) et ventrale (4) et dans la zone de chevauchement (38) des portions d'entrejambe (8) et dorsale (6), s'étendent dans le sens transversal (16), s'étendent parallèlement entre eux et sont espacés les uns des autres par des rubans non adhésifs (82), et dans lequel, ainsi, des structures (106) visuellement et/ou haptiquement perceptibles s'étendant dans le sens transversal (16) selon le tracé des rubans adhésifs (80) et des rubans non adhésifs (82) sont formées dans la face visible extérieure de l'article d'incontinence au niveau de la zone de chevauchement (36) des portions d'entrejambe (8) et ventrale (4) et au niveau de la zone de chevauchement (38) des portions d'entrejambe (8) et dorsale (6), et dans lequel un ruban adhésif (88, 90) plus large s'étendant dans la direction transversale (16) est prévu dans une zone marginale (84) de la zone de chevauchement (36, 38), qui est située du côté de la hanche dans la direction longitudinale (9), et/ou dans une zone marginale (86) de la zone de chevauchement (36, 38), qui montre dans la direction opposée à la hanche dans la direction longitudinale (9), la zone marginale (84) côté hanche respective et/ou la zone marginale (86) opposée à la hanche de la zone de chevauchement (36, 38) couvrant respectivement 20 % tout au plus de l'extension longitudinale de la zone de chevauchement (36, 38) respective.

2. Article d'incontinence selon la revendication 1, **caractérisé par le fait que** la largeur de rubans adhésifs (80), transversalement à leur extension, est comprise entre au moins 1 mm et 5 mm tout au plus, encore en particulier 4 mm tout au plus, encore en particulier 3 mm tout au plus et est, de préférence, de 2 mm.

3. Article d'incontinence selon la revendication 1 ou 2, **caractérisé par le fait que** la largeur des rubans non adhésifs (82), transversalement à leur extension, est comprise entre au moins 1 mm et 15 mm tout au plus, en particulier 10 mm tout au plus, en particulier 5 mm tout au plus et, encore en particulier, 3 mm tout au plus.

4. Article d'incontinence selon la revendication 1, 2 ou 3, **caractérisé par le fait que** la zone marginale (84) côté hanche respective et/ou la zone marginale (86) opposée à la hanche de la zone de chevauchement (36, 38) couvre respectivement 18 % tout au plus, en particulier 15 % tout au plus, en particulier 12 % tout au plus, en particulier 10 % tout au plus de l'extension longitudinale de la zone de chevauchement (36, 38) respective.

5. Article d'incontinence selon l'une quelconque des revendications précédentes, **caractérisé par le fait que** la largeur de rubans adhésifs (88, 90) marginaux, transversalement à leur extension, est d'au moins 5 mm, en particulier d'au moins 8 mm, en particulier d'au moins 12 mm, en particulier va jusqu'à 20 mm tout au plus, en particulier jusqu'à 16 mm tout au plus, en particulier jusqu'à 14 mm tout au plus.

6. Article d'incontinence selon l'une ou plusieurs des revendications précédentes, **caractérisé par le fait que** le rapport de la largeur de rubans adhésifs (80) à la largeur de rubans non adhésifs (82) immédiatement voisins est compris entre 0,2 et 3,0, en particulier entre 0,2 et 2,0, en particulier entre 0,2 et 1,5, en particulier entre 0,2 et 1,0, en particulier entre 0,4 et 0,8, en particulier entre 0,5 et 0,7.

7. Article d'incontinence selon l'une ou plusieurs des revendications précédentes, **caractérisé par le fait que** la largeur au moins des rubans adhésifs (80) qui sont situés à l'intérieur par rapport à des rubans adhésifs (88, 90) marginaux est respectivement égale.

8. Article d'incontinence selon l'une ou plusieurs des revendications précédentes, **caractérisé par le fait que** la largeur des rubans non adhésifs (82) est respectivement égale.

9. Article d'incontinence selon l'une ou plusieurs des revendications précédentes, **caractérisé par le fait que** la surface totale des rubans adhésifs (80, 88, 90), par rapport à la surface de la zone de chevauchement (36, 38) des portions d'entrejambe (8) et ventrale (4) ou bien des portions d'entrejambe (8) et dorsale (6), est comprise entre 35 et 60 %, en particulier entre 40 et 55 % et, encore en particulier, entre 40 et 50 %.

10. Article d'incontinence selon l'une ou plusieurs des revendications précédentes, **caractérisé par le fait que** le grammage du revêtement de colle dans les rubans adhésifs (80, 88, 90) est compris entre 2 et 20 g/m$^2$, en particulier entre 2 et 15 g/m$^2$, en particulier entre 2 et 10 g/m$^2$, en particulier entre 5 et 10 g/m$^2$, le grammage étant de préférence égal dans tous les rubans adhésifs.

11. Article d'incontinence selon l'une ou plusieurs des revendications précédentes, **caractérisé par le fait que** la surface de la zone de chevauchement (36) des portions d'entrejambe (8) et ventrale (4), par rapport à la surface de la portion ventrale (4), est d'au moins 12 %, en particulier comprise entre 15 et 40 %, en particulier entre 15 et 35 %, en particulier entre 15 et 30 %, en particulier entre 20 et 30 %, en particulier entre 22 et 30 %.

**12.** Article d'incontinence selon l'une ou plusieurs des revendications précédentes, **caractérisé par le fait que** la surface de la zone de chevauchement (38) des portions d'entrejambe (8) et dorsale (6), par rapport à la surface de la portion dorsale (6), est d'au moins 20 %, en particulier comprise entre 20 et 45 %, en particulier entre 20 et 40 %, en particulier entre 22 et 40 %, en particulier entre 25 et 40 %, en particulier entre 28 et 40 %.

**13.** Article d'incontinence selon l'une ou plusieurs des revendications précédentes, **caractérisé par le fait que** deux rubans adhésifs (88, 90) marginaux extérieurs sont prévus dans la zone de chevauchement (36) des portions d'entrejambe (8) et ventrale (4) et/ou dans la zone de chevauchement (38) des portions d'entrejambe (8) et dorsale (6), et une pluralité de rubans adhésifs (80) intérieurs est prévue entre celles-ci dans la direction longitudinale (9), la largeur des rubans adhésifs (88, 90) marginaux étant supérieure à la largeur des rubans adhésifs (80) intérieurs, en particulier étant au moins 4 fois, en particulier au moins 5 fois et, encore en particulier, 8 fois tout au plus, en particulier 7 fois tout au plus, plus grande que la largeur des rubans adhésifs intérieurs.

**14.** Article d'incontinence selon l'une ou plusieurs des revendications précédentes, **caractérisé par le fait qu'**au moins 8, en particulier au moins 10 rubans adhésifs (80, 88, 90) sont prévus dans la zone de chevauchement (36) des portions ventrale (4) et d'entrejambe (8), et au moins 15, en particulier au moins 18 et, encore en particulier, au moins 20 rubans adhésifs (80, 88, 90) sont prévus dans la zone de chevauchement (38) des portions dorsale (6) et d'entrejambe (8).

**15.** Article d'incontinence selon l'une ou plusieurs des revendications précédentes, **caractérisé par le fait que** les seconds moyens d'élastification (40, 42) s'étendent, suivant leur extension en direction de l'axe médian longitudinal (44) de l'article d'incontinence, à distance croissante les uns des autres en arc et en éventail.

**16.** Article d'incontinence selon l'une ou plusieurs des revendications précédentes, **caractérisé par le fait que** les premiers et/ou les seconds moyens d'élastification (28, 29; 40, 42) s'étendent parallèlement aux rubans adhésifs (80) dans la zone de chevauchement (36) des portions d'entrejambe (8) et ventrale (4) et dans la zone de chevauchement (38) des portions d'entrejambe (8) et dorsale (6).

Fig. 1

Fig. 2a

Fig. 2b

Fig. 4

Fig. 3

Fig. 5

EP 2 849 694 B1

Fig. 6

Fig. 11

Fig. 7

Fig. 8

EP 2 849 694 B1

Fig. 9

Fig. 10a

Fig. 10b

Fig. 10c

**EP 2 849 694 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007056126 A1
- EP 2241296 A1
- WO 2011055546 A1
- EP 2377500 A1
- DE 102007055628 A1
- US 20040116886 A1
- US 7250549 B
- US 7591810 B2